# EUROPEAN PATENT SPECIFICATION

(11) **EP 2 157 918 B1**
(45) Date of publication and mention of the grant of the patent: **11.06.2014**
(21) Application number: 08755318.6
(22) Date of filing: 12.05.2008
(51) Int. Cl.: A61B 17/064, A61B 17/068, A61B 17/10, A61B 17/28, A61F 5/00, A61B 17/30

(54) **DEVICES FOR STOMACH PARTITIONING**
VORRICHTUNGEN ZUR ABTEILUNG DES MAGENS
DISPOSITIFS POUR RÉALISER UNE PARTITION DE L'ESTOMAC

(30) Priority: 12.05.2007 US 917644 P
(43) Date of publication of application: 03.03.2010
(73) Proprietor: Barosense, Inc., Redwood City, CA 94063 (US)
(72) Inventor: BALBIERZ, Daniel, J., Redwood City, CA 94061 (US); COLE, Dave, San Mateo, CA 94403 (US); CREWS, Samuel, T., Woodside, CA 94062 (US); SWOPE, Brett, Gaithersburg, MD 20882 (US)
(74) Representative: Asmussen, Arnold
(86) International application number: PCT/US2008/063440
(87) International publication number: WO 2008/141288

(56) References cited:
- WO-A-2005/037152
- WO-A-2007/041598
- FR-A- 2 768 324
- US-A- 5 403 326

## Description

### BACKGROUND OF THE INVENTION

Surgical procedures used to modify the shape and/or size of a stomach are effective in reducing weight and resolving associated co morbidities. Unfortunately these surgical procedures are invasive and are associated with high levels of peri-operative and post operative complications.

Some procedures have been introduced which utilize natural body orifices for surgery to reduce the invasiveness of these procedures. Natural orifices include, but are not limited to the esophagus, anus and vagina. These procedures are less invasive by nature but have limitations as will be described below.

Natural orifice procedures have largely been directed at the Gastrointestinal (GI) Tract, but also include procedures which exit the GI tract, and perform surgeries normally done laparoscopically. Access to the peritoneal space for example can be accomplished by penetrating the stomach wall.

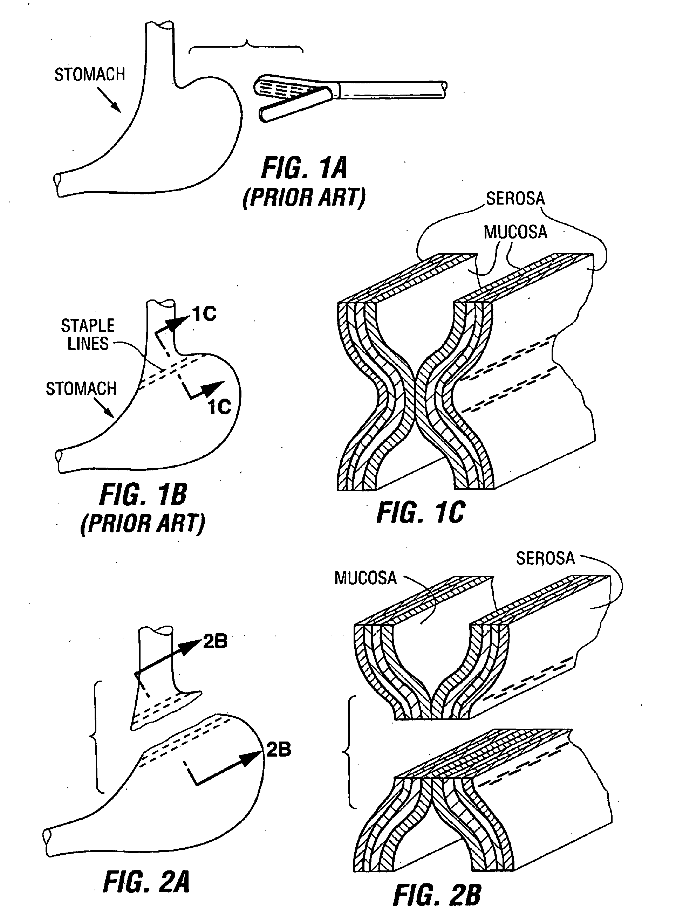

One primary means of stomach modification is by the use of surgical or laparoscopic staplers. These devices are able to surgically or laparoscopically appose multiple layers of tissue and connect them by use of multiple staple rows. Early procedures stapled across the outside of the stomach, which brought the mucosa of two sides of the stomach into apposition. (Figs. 1A-1C) There was, and is, a high rate of failure of these staple lines due to the nature of the GI tract. Staple line dehiscence was common and resulted in inadequate clinical results. The solution was to surgically staple the tissue and cut between the staple lines. This enabled edge to edge healing to occur, and provided for a robust tissue bridge. (Figs. 2A - 2B) The separation/cutting of tissues is now common for surgical procedures such as Roux-En-Y Gastric Bypass, Sleeve Gastrectomy, and Vertical Banded Gastroplasty. However, less invasive procedures allowing stomach partitioning using natural orifice access are highly desirable. Other devices and methods for modifying stomach tissue, including fastening and/or cutting tissue, are shown and described in published PCT Application WO 2005/037152.

FR 2 768 324 A discloses a surgical instrument enabling two zones of soft tissue that are normally mutually distant to be percutaneously joined together. The instrument comprises at least one tube that can be introduced percutaneously into the body of the patient until its distal extremity is situated in the proximity of the two zones of tissue to be joined, two elongated members arranged in the tube, of which each comprises a distal extremity suitable for equipping one of the two tissue zones to be joined. Moreover, the instrument comprises means for displaying the distal end portions of the elongated members between a mutually close position, in which the portions do not hinder the introduction of the tube and enabling the tissue zones, once gripped, to be brought together, and a mutually distant position, in which each of the distal extremities is able to grip the corresponding tissue zone. Further, the instrument has at least one coupling member, provided with coupling means for each tissue to be joined, the coupling member enabling the two tissue zones to be fixed to each other once brought together by the displacement of the distal end portions to the mutually close position. The instrument also has a stem linked to each coupling member, which can be manipulated from outside the patient's body to displace axially the coupling member, wherein the stem is separable from the coupling member, and an abutment forming member, contained in the tube or tubes, enabling axial immobilization of each coupling member when traction is applied to the stem so as to press the coupling member against the abutment forming member, until separation of the stem from the coupling member is achieved.

Some existing procedures attempt to partition the stomach from the inside by connecting tissue within the stomach. To date these procedures have demonstrated a high failure rate. Improved devices and methods for creating robust stomach partitions using natural orifice access would be beneficial.

Another problem with current stapling procedures is they are permanent in nature, or designed to be so. In a Roux en Y Gastric Bypass, no provision exists for reversing the procedure. If a patient wished to return to his normal stomach function, it would be impossible to do so. Thus it would also be beneficial to have a procedure that was reversible, also by means of a natural orifice.

Tools as designed and described on the following pages address both deficiencies of current technology.

### BRIEF DESCRIPTION OF THE DRAWINGS

Figs. 1A - 1B schematically illustrate a prior art stomach partitioning method. Fig. 1C illustrates two section of stomach wall tissue joined according to the method of Figs. 1A and 1B.
Fig. 2A schematically illustrates a prior art stomach partitioning method. Fig. 2B illustrates two section of stomach wall tissue joined and transected according to the method of Fig. 2A.
Fig. 3A is a perspective view of a partitioning tool.
Fig. 3B schematically shows the staple holder of the partitioning tool of Fig. 3A.
Figs. 4A - 4C are plan views showing three examples of suitable staple holders.
Fig. 5A is a plan view of the partitioning tool of Fig. 3A with the arms expanded.
Fig. 5B is an end view of the partitioning tool of Fig. 5A.
Fig. 5C shows the partitioning tool of Fig. 3A with the arms extended for streamlined advancement of the tool through the esophagus into the stomach.
Figs. 5D and 5E are similar to Figs. 5A and 5B but show a modification in which the arms are spaced by an alternate angle.
Fig. 6A is a perspective view of the partitioning tool illustrating extension of the graspers along intersecting paths.
Fig. 6B is a perspective view of the partitioning tool illustrating extension of the graspers along parallel paths.
Figs. 6C - 6D are perspective views of the distal shaft and stapler head of an alternative embodiment in which the direction of relative movement of the stapler head and anvil is transverse to the orientation of the distal portion of the shaft.
Figs. 7A - 7G illustrate various forms of graspers.
Fig. 7H is a plan view of a staple holder and illustrates a grasper drawing tissue across the surface of the staple holder.
Figs. 7I- 7K are similar to Fig. 7H and show alternative staple holder shapes and grasper arrangements.
Figs. 8A - 8F are a series of drawings illustrating a first exemplary method of using the disclosed partitioning system.
Figs. 9A - 9F are a series of drawings illustrating a second exemplary method of using the disclosed partitioning system.
Figs. 9G - 9J are a series of drawings illustrating use of a modified partitioning system.
Figs. 9K - 9N are a series of drawings illustrating use of yet another modified partitioning system.
Figs. 9O - 9P illustrate an alternate of using the disclosed partitioning system.
Figs. 9Q - 9S disclose exemplary methods for advancing the graspers in the method of Figs. 9O - 9P.
Fig. 10A is a plan view of a stomach illustrating a stomach wall partition formed in the stomach wall tissue.
Figs. 10B - 10C illustrate a first orientation of plications forming a partition or tissue structure within the stomach.
Figs. 10D - 10F illustrate a second orientation of plications forming a partition or tissue structure within the stomach.
Figs. 11A - 11E illustrate various arrangements of plications to form plications in a stomach.
Figs. 11F - 11J illustrate formation of a partition by forming tissue plications around the shaft of an instrument disposed in the stomach.
Fig. 12 schematically shows a stomach and illustrates various partition locations that can be formed by creating partitions or tissue structures as disclosed herein.
Figs. 13A - 13E are a sequence of steps illustrating reversal of a stomach partition.
Figs. 14A -14D illustrate the use of plugs to close gaps between plications formed in tissue or to close cut holes formed in plications.
Figs. 15A through 15D illustrate the use of plugs within holes cut into plications to hold two or more two-layer plications together, such as to form a partition.
Figs. 16A and 16B illustrate the use of plugs positioned within holes cut into plications to restrict flow of food towards the distal stomach
Fig. 17 illustrates plug designs having anchoring features to restrain the anchors within holes through tissue.
Fig. 18 illustrates heads or lids for plugs.
Fig. 19A is a perspective view of an alternative partitioning tool.
Fig. 19B is a plan view of the jaws of the partitioning tool of Fig. 19A.
Fig. 19C shows the jaws of the partitioning tool of Fig. 19A in the closed position.
Fig. 19D is a perspective view illustrating use of the grasper to draw tissue through the partitioning tool of Fig. 19A, and use of the partitioning tool for tissue compression, stapling and optioning cutting.
Fig. 19E illustrates a partition formed in a stomach using multiple plications formed according to the exemplary method shown in Fig. 19D.
Figs. 20A - 20G illustrate use of a system comprising an alternate grasper and the partitioning tool of Fig. 3A to close an opening in a body wall.
Figs. 21A - 21D show a stapler head of a partitioning tool and illustrate articulation and rotation features.
Figs. 21E - 21G illustrates use of the articulation and rotation features of Figs. 21A - 21D to access tissue within a stomach.

### DETAILED DESCRIPTION

The present application describes a device for forming tissue structures within, remodeling, or partitioning a body cavity, hollow organ or tissue tract. The application will discuss the device and an exemplary method in connection with use in the stomach for formation of plications such as for stomach partitioning or other purposes, although they may be used for applications other than stomach remodeling or partitioning.

When an area of the stomach wall is drawn inwardly (bringing a two-layer "pinch" or fold of tissue toward the stomach exterior), corresponding regions of serosal tissue on the exterior of the stomach are positioned facing one another. According to a preferred exemplary method disclosed herein, two or more such areas or pinches of the stomach wall are engaged/grasped and drawn inwardly using instruments passed into the stomach via the mouth. The two or more pinches of tissue are held in complete or partial alignment with one another as staples or other fasteners are driven through the pinches, thus forming a four-layer tissue plication. Over time, adhesions formed between the opposed serosal layers create strong bonds that can facilitate retention of the plication over extended durations, despite the forces imparted on them by stomach movement. A cut or cut-out may be formed in the plication during or separate from the stapling step to promote edge-to-edge healing effects that will enhance tissue knitting/adhesion and will ultimately contribute to the durability of the plication, despite the fact that mucosal tissue of one tissue pinch is positioned in apposition with the mucosal tissue of the other tissue pinch.

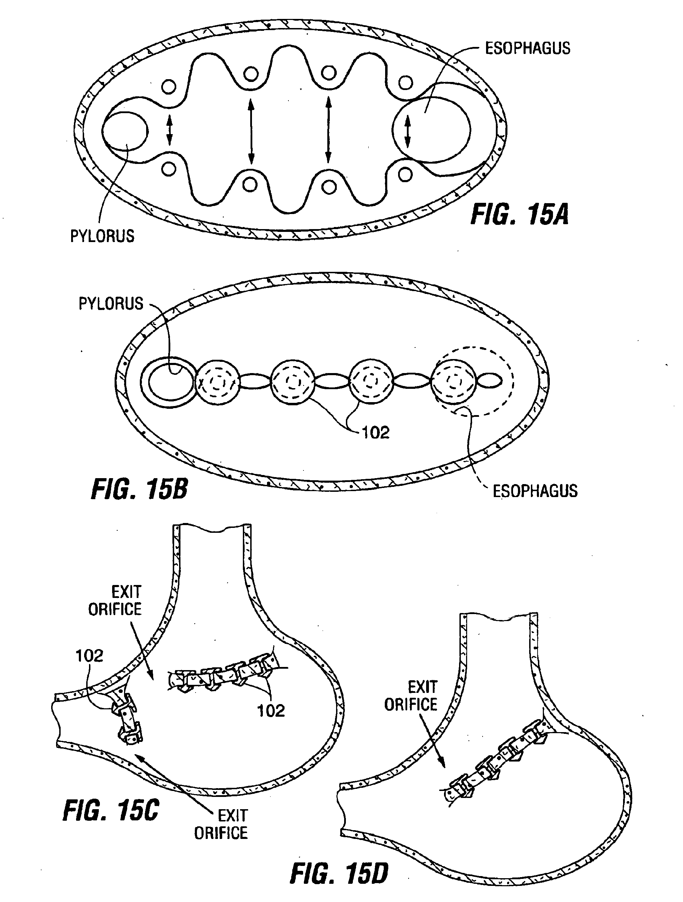

One or more such plications may be formed for a variety of purposes. For example, plications may be used to induce weight loss by creating a barrier or narrowing within the stomach that will restrict the flow of food from the proximal stomach towards the distal stomach. For example, a partition or barrier may be oriented as in Fig. 15C or 15D to extend across the stomach, leaving only a narrow exit orifice through which food can flow from the proximal stomach to the distal stomach, or a similar antral barrier (Fig. 15C) may be formed that will slow stomach emptying of stomach contents into the pylorus. In other cases, partitions or plications may be used to form a proximal pouch in the stomach or to reduce stomach volume to cause sensations of fullness after a patient eats relatively small quantities. Plications might also be used as a treatment for GERD to create a shield between the stomach and esophagus that will minimize reflux. Plications might also be used to close perforations in the stomach wall.

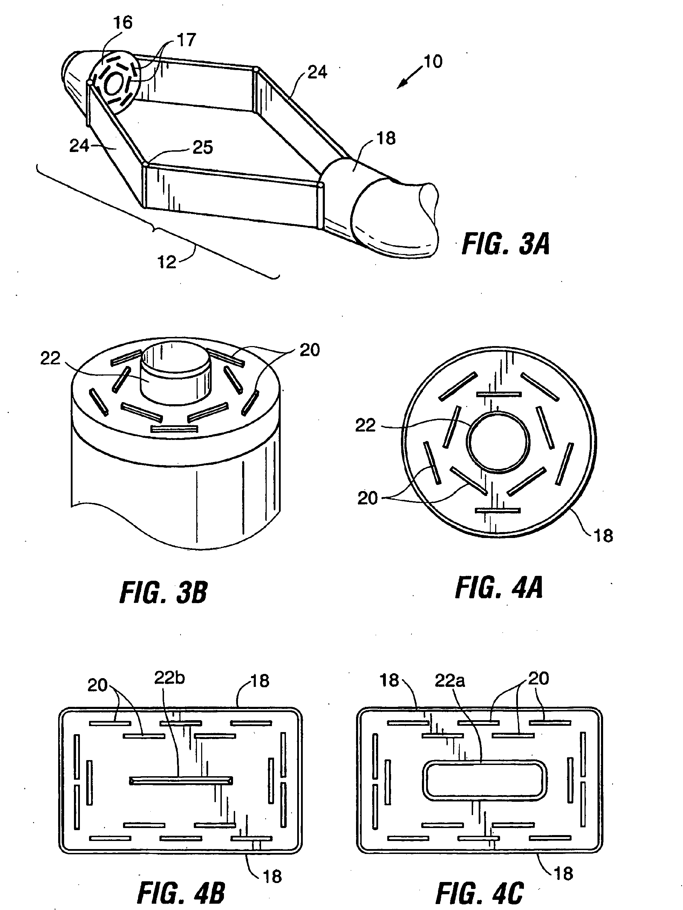

Referring to Fig. 3A, an exemplary embodiment of a partitioning system includes a partitioning tool 10. Although the partitioning tool may include various mechanisms for applying a fastening element (e.g. clips, sutures, staplers etc.) to tissue, the disclosed embodiment utilizes a stapler head 12 positioned at the distal end of an elongate shaft 14. The shaft is of sufficient length to allow it to be advanced into the target body cavity (e.g. stomach) through a natural orifice (e.g. the mouth). Stapler head 12 includes an anvil 16 having recesses 17 for holding staples, and a staple holder 18 (Fig. 3B). In the preferred embodiment, the staple holder is a removeable/replaceable cartridge and/or it may be refillable by inserting additional staples into it. In other embodiments, the staple holder may be neither replaceable nor refillable.

A preferred stapler is a circular stapler which preferably contains multiple concentric rows of staples 20, surrounding a circular cutter 22. The cutter is not mandatory, and can be omitted if cutting of tissue is not desired. Applications for a plication tool that lacks a cutting element might include those involving the creation of a simple plication (e.g. a single pinch rather than a plication formed of two tissue pinches), apposition of multiple tissue layers, the closing of a stomach wall perforation, or the resection of a targeted tissue (i.e. appendix), etc.

While a circular staple arrangement may be preferable other configurations of staples are also suitable. The staples can be positioned to surround a central cutter of any shape when one is included. Suitable cutter shapes include the round cutter 22 of Fig. 4A, the rectangular cutter 22a Fig. 4C, a linear cutting blade 22b of the type shown in Fig. 4B, or an oval cutter etc.. The cutter can be configured to simply cut layers of tissue (e.g. see the cutting blade 22b of Fig. 4B) or to remove a portion of tissue internal to the cutter (see the punch-type cutters 22, 22a of Figs. 4A and 4C).

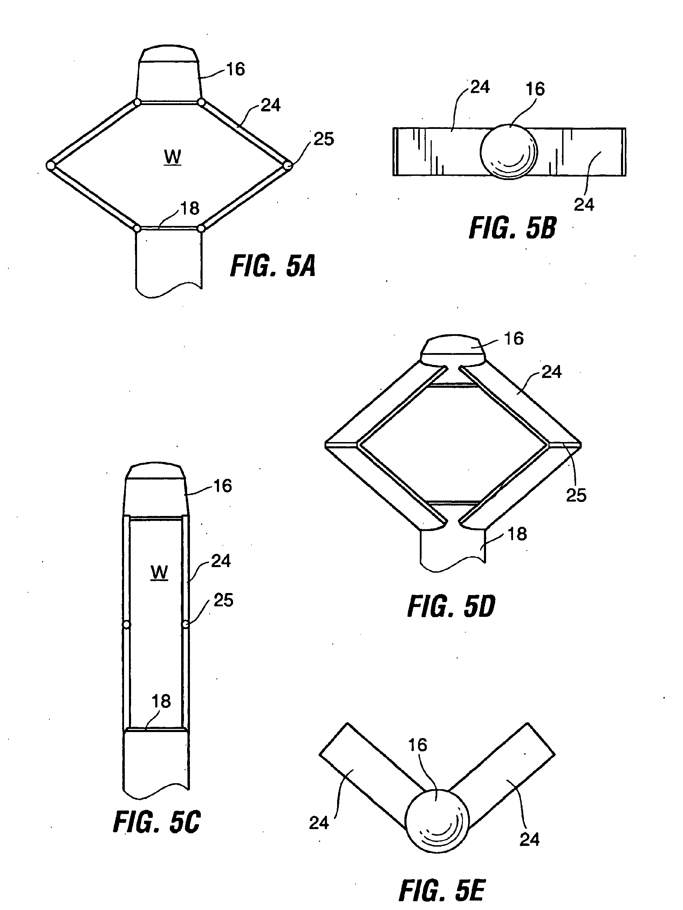

The staple holder 18 and anvil 16 are connected by an arm assembly having collapsible arms 24. The arms are collapsed into a generally elongate position (Fig. 5C) for insertion into the lumen of the body, but are opened to the expanded position (Fig. 5A) once in a hollow organ or tract. Moving the arms to the expanded position moves the stapler holder and anvil relatively towards one another while increasing the lateral dimension of the window W bounded by the arms 24, staple holder 18 and anvil 16. This motion can be continued following tissue acquisition to compress the acquired tissue between the staple holder and anvil. In the illustrated examples, the arms 24 include proximal and distal sections coupled by hinges 25. In these embodiments, the arms are pivoted relative to the hinges for expansion and collapse of the arms. The arms can be linear, curved or oriented at varying angles relative to the staple cartridge. Moreover, the arms 24 can be spaced by an angle of 180° as shown in Fig. 5B, or by a larger/smaller angle as shown in Fig. 5E.

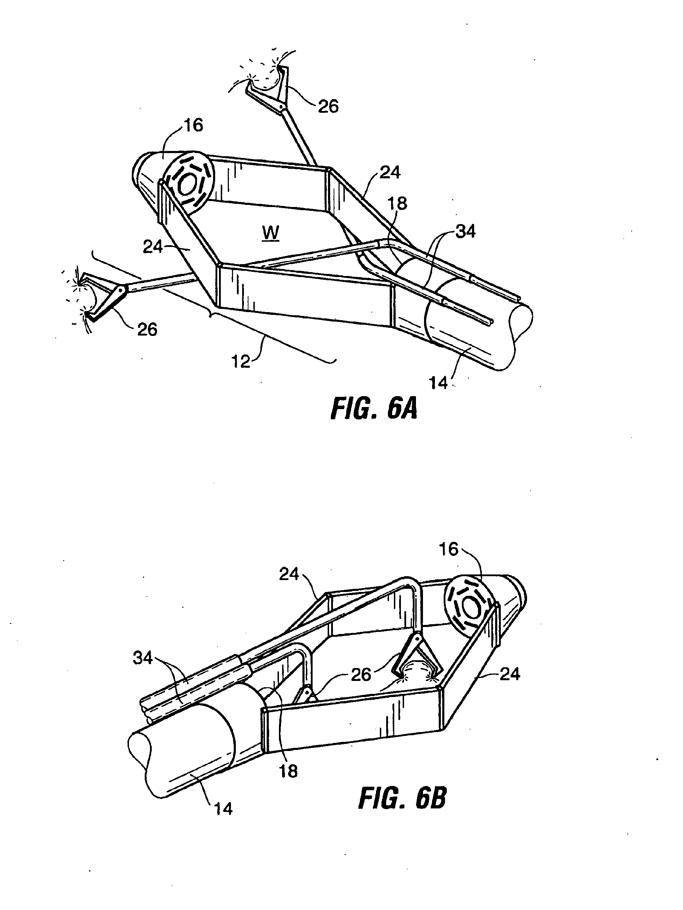

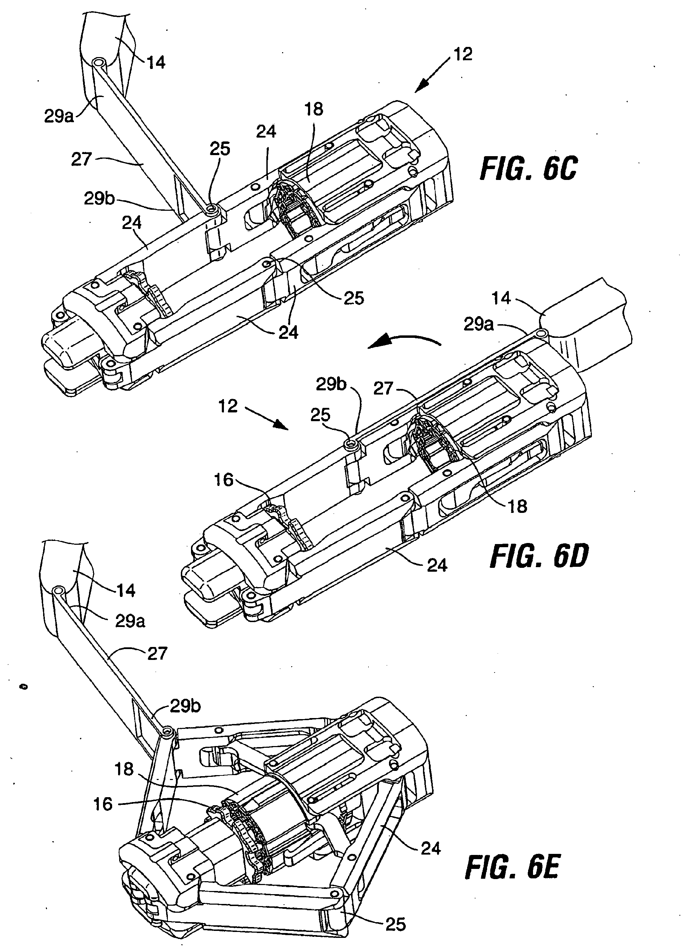

Referring to Fig. 6A, coupled to or provided with the stapler are one or more, preferably two, three or more, tissue acquisition devices, which will also be referred to as "engagers" or "graspers" 26 which are designed to engage tissue and draw the tissue into position between the stapler anvil and cartridge. In the embodiments shown in Figs, 6A and 6B, the graspers are positioned to pass from one side of the "window" bounded by the stapler arms, through the window, and used to grasp tissue on the opposite side of the window. These graspers are then withdrawn back through the window to draw the grasped tissue between the cartridge and anvil. In other embodiments, the arms can engage tissue and draw it between the cartridge and anvil without necessarily passing through the window. Such embodiments include those similar to the Fig. 5E embodiment, in which the arms are oriented angularly relative to one another when viewed along the longitudinal axis of the device shaft.

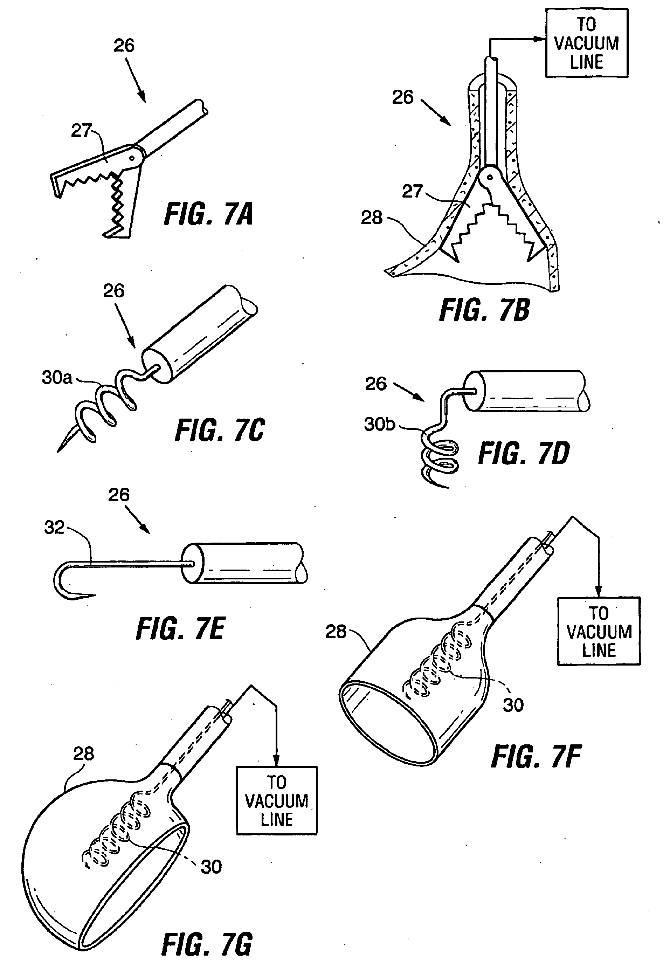

Referring to Figs. 7A - 7G, the graspers can be simple alligator or forceps type graspers 26, vacuum chambers 28, corkscrews which can be traditional corkscrews 30a or gear-driven perpendicular cork-screws 30b, hooks 32, or any combination thereof, such as a corkscrew 30 in combination with a vacuum chamber as shown in Fig. 7B. Fig. 7G shows corkscrew 30 longitudinally advanceable within a vacuum chamber 28 having a side facing opening, such that tissue can be drawn into vacuum chamber using suction, and such that the corkscrew 30 can then be moved in a longitudinal direction and screwed into the tissue within the vacuum chamber. A similar design using a longitudinally advanceable barb rather than a corkscrew may also be configured. Other alternative designs which are capable of acquiring the targeted tissue may instead be used.

In this disclosure, the term "grasper" is used to refer generally to any type of tool that can be used to engage or acquire tissue via any means (grasping, hooking, penetration, suction, adhesion, etc.) so the acquired tissue can be positioned between the staple holder and anvil. Similarly, even though some of the disclosed graspers do not physically "pinch" tissue, the term "pinch of tissue" may be used in this disclosure to refer to a fold, area, or tab of tissue acquired using a grasper for positioning of that fold, area ,or tab between the staple holder and anvil.

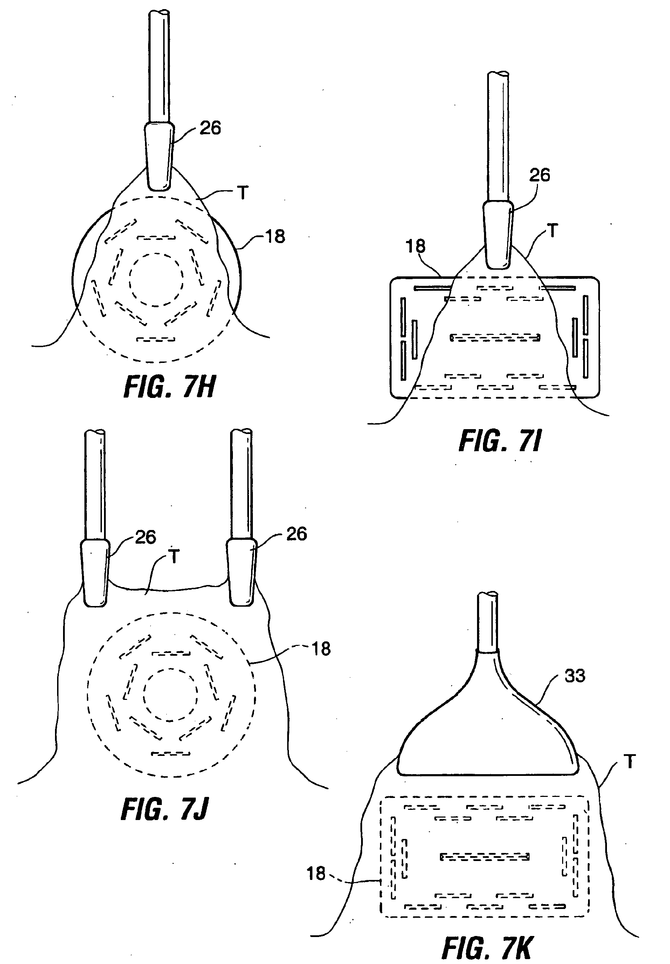

Figs. 7H and 7I schematically illustrate use of a grasper 26 to draw acquired tissue into position between a staple holder 18 and an anvil (not shown). If the tissue that can be acquired by a single grasper 26 lacks the width to extend fully across the staple holder or anvil as shown, an alternate grasper arrangement may be used in which each individual grasper is replaced by a pair of side-by-side graspers 26 as shown in Fig. 7J, or by a type of grasper that can acquire a broader area of tissue, such as the Alice clamp-type grasper 33 shown in Fig. 7K. Doing so can ensure that each acquired piece of tissue can be positioned to receive the full array of staples from the stapler holder. These arrangements give the pinch of tissue a leading edge that is more rectangular, as opposed to the more triangularly shaped pinch created using a single grasper.

The graspers need not be integral to the stapler but could be separate tools used in conjunction with the stapler. In use of the Fig. 6A and 6B embodiments, the graspers are advanced to the target tissue site through guide tubes 34 on the stapler shaft 14, under direct visualization or fluoroscopy. Alternatively or additionally, the device shaft itself can be articulated to bring the tools into contact with the desired tissue. The guide tubes 34 may include articulation features (e.g. pullwires) to facilitate positioning of the graspers. The tissue graspers can additionally be equipped with articulation means to aid in grasper positioning and/or withdrawal of tissue into position between the cartridge and anvil.

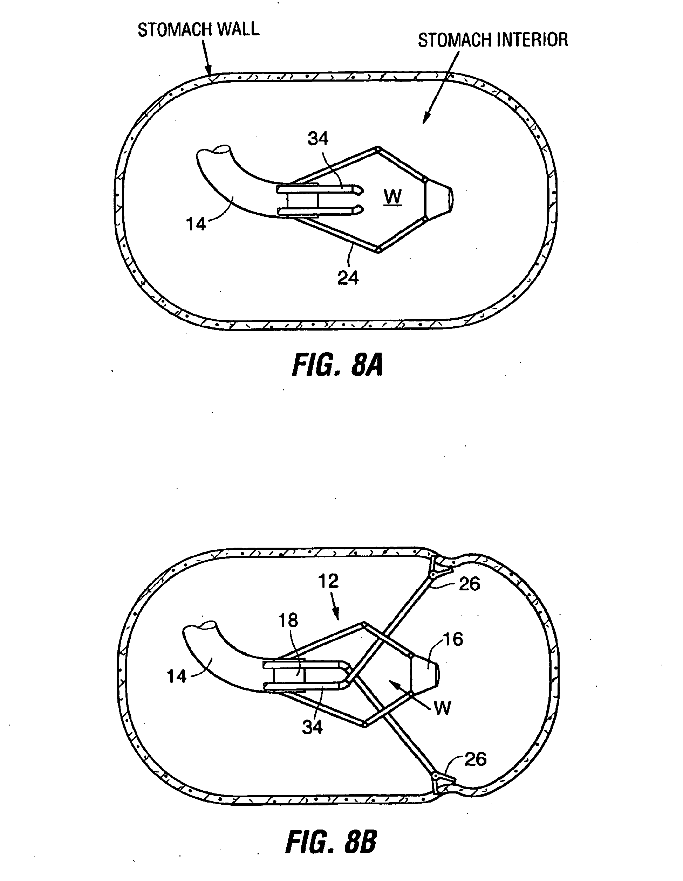

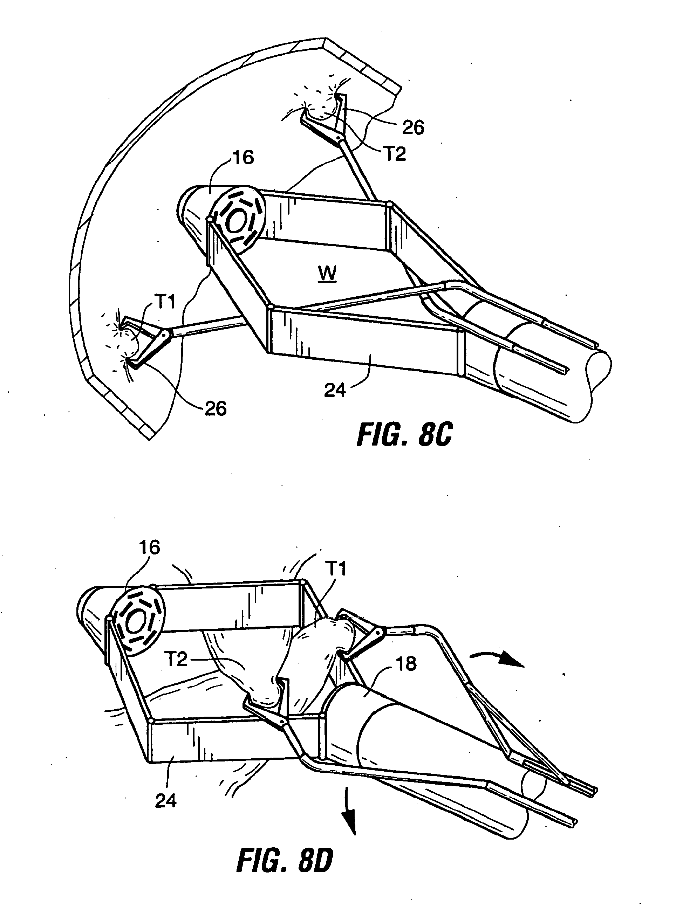

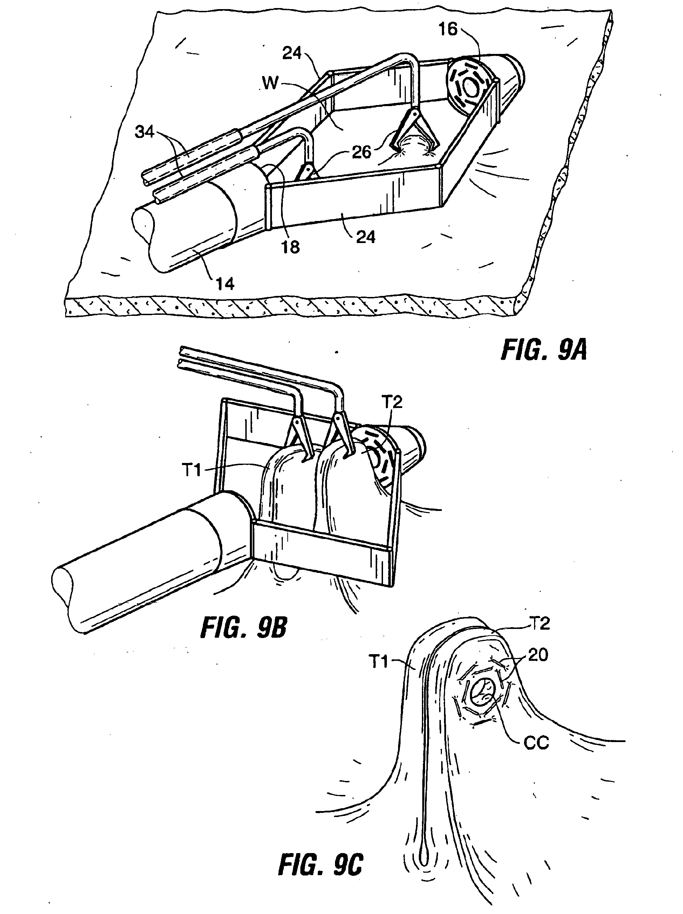

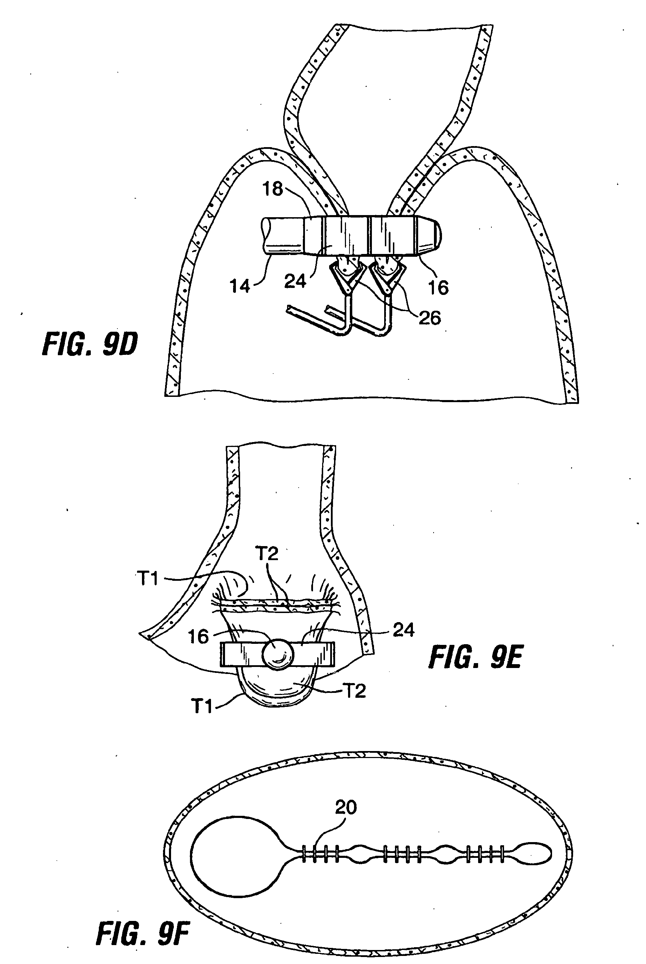

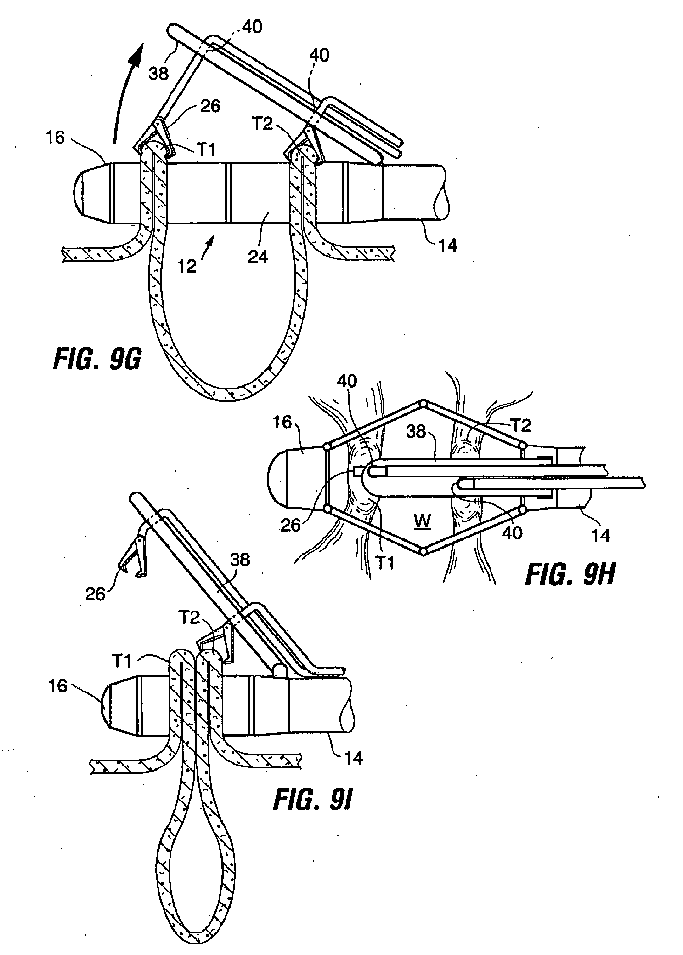

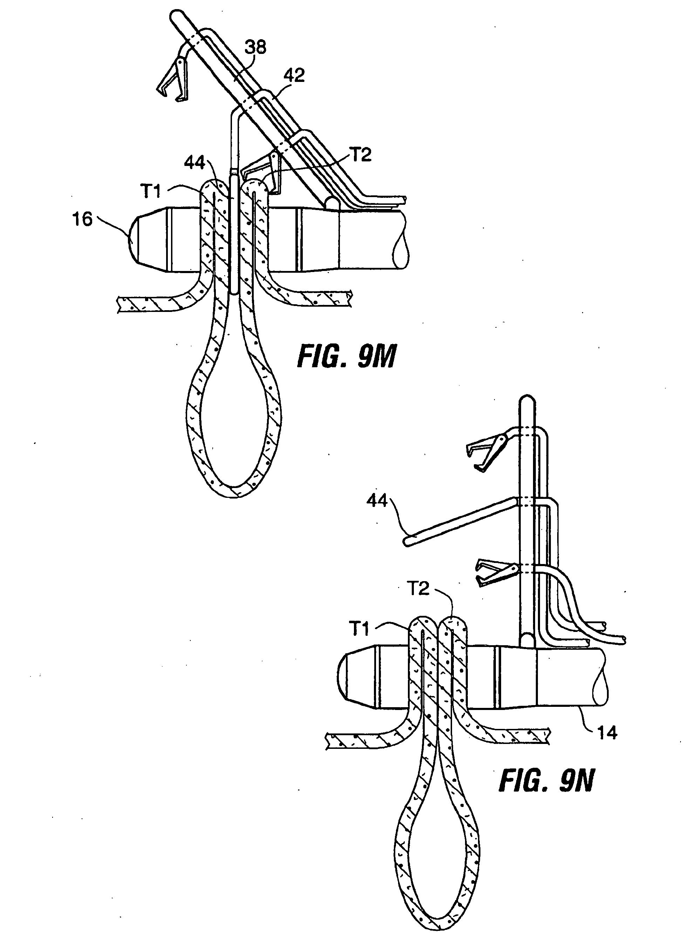

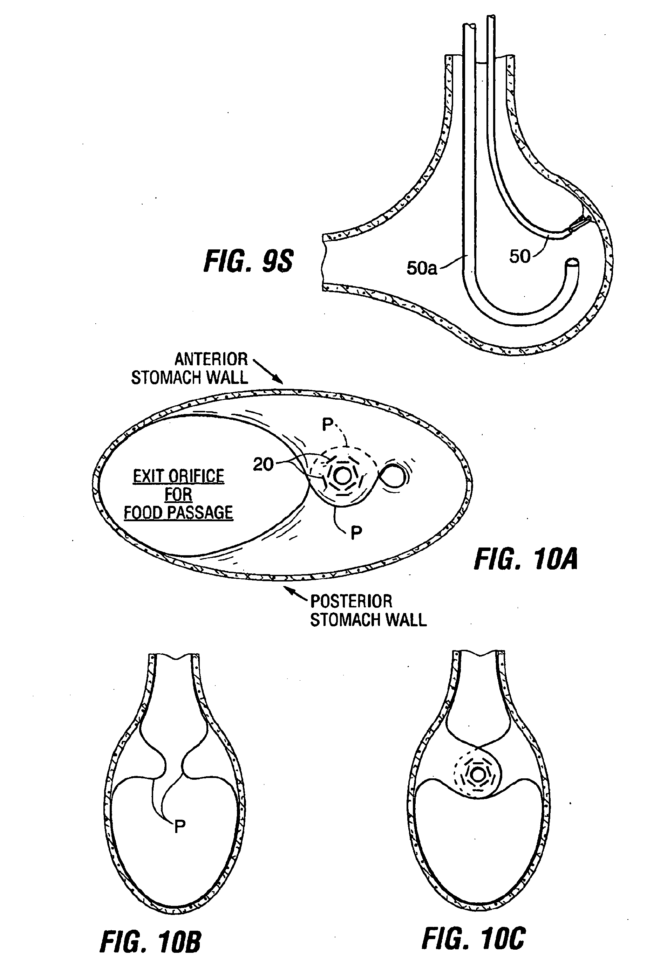

Grasping tools are passed from one side of the window W created by the arms 24 of the device. In the Fig. 6A embodiment, the graspers 26 extend non parallel to each other such that when retracted, the tissue acquired by each grasper overlaps the tissue acquired by the other grasper as illustrated in the sequence of steps shown in Figs. 8A - 8E. Graspers can instead be positioned parallel to one another and staggered slightly as in the Fig. 6B embodiment. This configuration allows tissue to be pulled in a relatively perpendicular orientation to the stapler head as illustrated in the sequence of steps shown in Figs. 9A - 9C. In either case, when desired tissues are acquired and are positioned between the cartridge and anvil of the stapler, the device is activated thereby compressing tissue, firing staples, and in the preferred embodiment, cutting a central piece of tissue bounded by the staple lines. This cut enables the body to duplicate the strong edge to edge healing which occurs in durable surgical procedures.

Several of Applicants' prior applications include embodiments of tissue plicators having features that may be used in the partitioning tool 10 to effect tissue compression, stapling firing, staple reinforcement, and/or cutting. In particular, U.S. Application No. 11/542,457 (U.S. 2007-0219571), Endoscopic Plication Device and Method, filed October 3, 2006, U.S. Application No. 11/900,757 (US-2008 190989), Endoscopic Plication Device and Method, filed September 13, 2007, and/or U.S. Application No.12/050,169 (US-2009 236400), Endoscopic Stapling Devices and Methods, filed March 18, 2008, disclose mechanisms for achieving tissue compression (using hydraulics or other means) by decreasing the relative separation between the staple holder and anvil, for hydraulically driving staples, for articulating the stapler head, for cutting tissue, and for reinforcing staple lines with buttressing material.

One exemplary method of tissue remodeling in accordance with the disclosed embodiments will next be described in connection with Figs. 8A through 8F. In preparation for use of the device, the stapler head 12 is positioned in the collapsed position shown in Fig. 5C and the stapler head is advanced through the mouth and esophagus into the stomach (Fig. 8A). The proximal end of the shaft 14 remains outside the body.

Next, the tissue graspers 26 are passed through the guide tubes 34 on the stapler shaft. The graspers are passed from one side of the shaft, through the window W defined by the stapler arms 24, and used to grasp regions of stomach wall tissue as shown in Figs. 8B and 8C. The stapler head 12, shaft 14, guide tubes 34, and/or graspers 26 can be manipulated (e.g. deflected, articulated or rotated) to reach the desired tissue. As illustrated in Fig. 8B, in this embodiment the graspers are oriented to cross each other between the staple holder 18 and anvil 16. This causes the grasped pinches of tissue T1, T2 to overlap one another as shown as the graspers are withdrawn or manipulated to draw the engaged tissue between the staple holder (cartridge) and anvil. As discussed in connection with Figs. 7F - 7I, each of the individual graspers 26 may be replaced with a pair of spaced-apart graspers, so as to acquire a broader pinch of tissue.

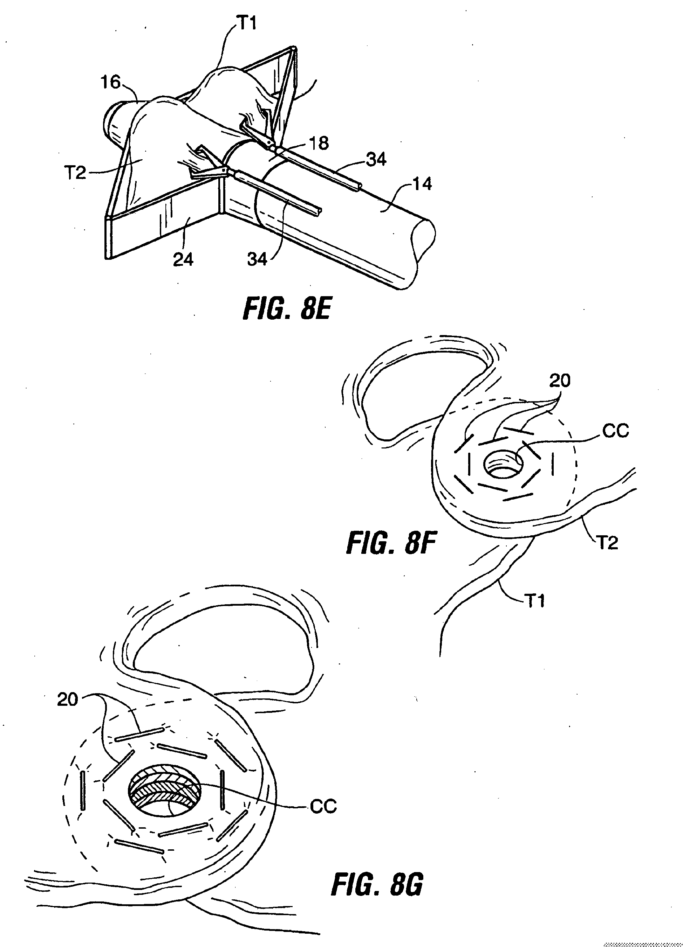

The pinches of tissue are compressed between the staple holder and anvil, and staples are driven through the pinches of tissue as shown in Fig. 8E. In a preferred form of device, at least two concentric rings of staples 20 are formed through the tissue, with a central core CC cut through the tissue by the cutter 22. The plicated tissue (Fig. 8F) is released from the tool 10, and the arms are pivoted to the elongated position of Fig. 5C. As best seen in Fig. 8G, the plication comprises four layers of tissue (two from each tissue pinch), with the staples and the cut extending through all of the four layers. The plications may be reinforced using reinforcing rings and/or buttressing materials or substances as disclosed on Applicants' prior applications referred to above.

Referring to Figs. 6C - 6E, in still another embodiment, the staple head 12 is mounted to the shaft in a position that is rotated (e.g. 90°) from the position shown in Fig. 6A. In other words, the staple holder and anvil may be moveable relatively towards one another in a direction that is transverse to the distal portion of the shaft, rather than generally parallel to the shaft. For example, the staple head may be coupled to a pivot member 27 having a first end 29a pivotally coupled to the shaft 14, and a second end 29b pivotally coupled to the staple head at the hinge 25 of one of the arms 25. For insertion into the body, the stapler head 12, pivot member 27, and shaft 14 extend generally longitudinally as in Fig. 6D. Once the stapler head 12 is in the stomach, the stapler head 12 is pivoted to a position generally transverse to the shaft 14. Optimal stapler head position is achieved by pivoting the stapler head relative to the pivot member 27 and/or pivoting the pivot member 27 relative to the shaft 14. As with other embodiments, the arms 24 are pivoted relative to the hinges 25 to decrease the relative separation between the anvil 16 and staple holder 18 for tissue compression. Figs. 6C - 6E include additional features useful for tissue compression and staple firing which are disclosed in greater detail in U.S. Application No. 12/050,169 (US-2009 236400), Endoscopic Stapling Devices and Methods, filed March 18, 2008 (Attorney Docket No. BARO-1900).

In other embodiments, the partitioning tool may be equipped to reorient the graspers as they withdraw the pinches of tissue towards the window, so as to ensure the pinches are properly aligned with one another and with the staple housing and anvil.

Figs. 9A through 9F illustrate a second exemplary method for forming a plication in the stomach. This method is largely similar to that shown in Figs. 8A through 8E, but differs in that it is performed using the configuration of Fig. 6B in which the graspers 26 extend parallel to one another. As discussed in connection with Fig. 7F - 7I, each one of the parallel graspers 26 may be replaced with a pair of side-by-side graspers to allow acquisition of a broader pinch of tissue. As shown in Fig. 9A, the parallel graspers 26 are passed through the window W of the stapler head 12 and used to engage regions of tissue. The regions or pinches of tissue T1, T2 are withdrawn through the window W as shown in Figs. 9B, 9D and 9E, and the device is activated to compress and staple the pinches to form a plication, and to preferably form a cutout CC surrounded by the rings of staples 20.

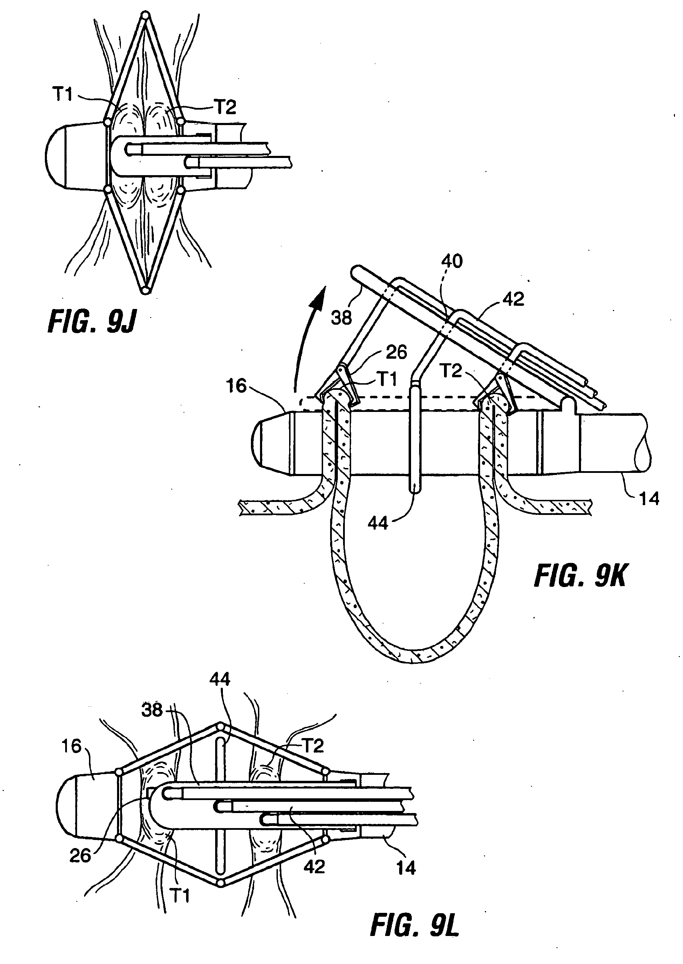

Figs. 9G - 9J show a modification to the Fig. 9A embodiment in which the graspers 26 are coupled to a pivot member 38. Pivot member 38 may be a plate pivotally coupled to the stapler head 12 at its proximal end. The graspers extend through holes 40 in the pivot member. Once the graspers have engaged pinches of tissue T1, T2, the pivot member is pivoted away from the window W, carrying the graspers and the acquired tissue and thereby drawing the pinches of tissue through the window. As with prior embodiments, the stapler is activated to compress the tissue pinches between the cartridge and anvil, and to drive staples through the compressed tissue (Figs. 9I and 9J).

In a further modification shown in Figs. 9K - 9M, an elongate support 42 having a partition 44 extends through a hole 40 in the pivot member 38 such that it extends through the window W. When tissue pinches T1, T2 are acquired by the graspers, the partition is disposed between the pinches. (Figs. 9K and 9L) Compression of the pinches between the cartridge and anvil sandwiches the partition 44 between the pinches. The partition is formed of a flexible material that is less slippery than the surface of the tissue. Its presence during compression will minimize the likelihood that the slippery nature of the tissue will cause one of both of the pinches T1, T2 to slip or "squirt" out of alignment with the staple holder/anvil before or during compression.

The partition 44 may be retracted between the steps of tissue compression and tissue stapling by pivoting the pivot member away from the window W as shown in Fig. 9N. Alternatively, the partition 44 may be cut or released from its support (prior to or after staple firing), leaving it in place between the tissue pinches. According to this latter exemplary method, staples advanced through the tissue pinches pass through the partition 44, maintaining the position of the partition. For this embodiment, the partition may be formed of a material that will absorb, degrade, or erode within the body over a period of time.

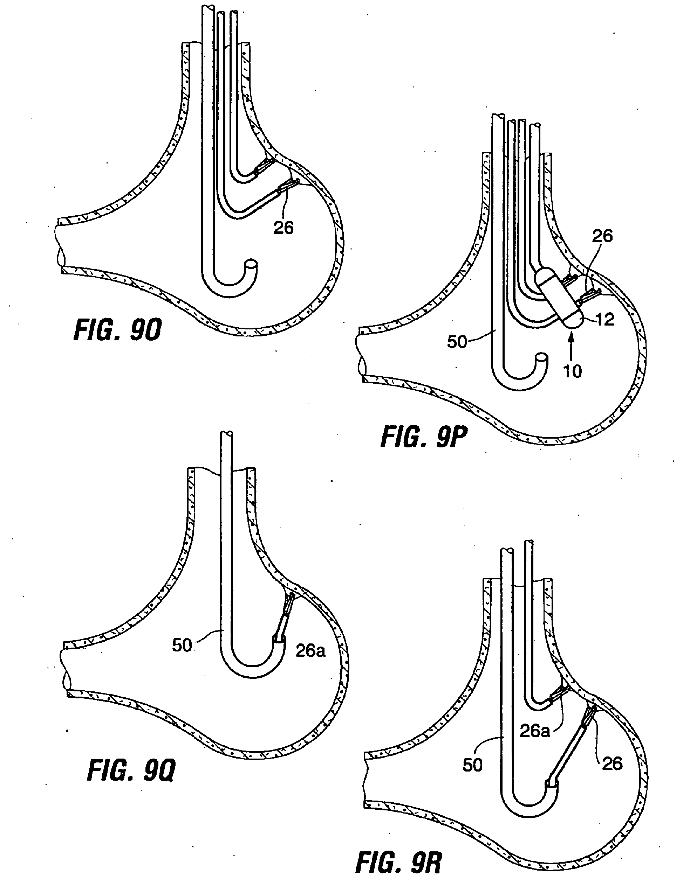

Figs. 9O and 9P illustrate another alternative system in which the graspers 26 are used to engage stomach wall tissue, and in which the window of the partitioning tool 10 is slipped over the proximal ends of the graspers (outside the body) and guided into the stomach over the shafts of the graspers. As illustrated in Figs. 9Q - 9S, during this procedure, an articulating grasper 26a (Fig. 9R) may be used, and one or both of the graspers 26 may be passed down the channel of an articulating endoscope 50. A separately positioned endoscope 50a may be positioned independent of the graspers and retroflexed to allow visualization of the grasping and stapling steps.

Once the partitioning tool has been advanced into position over the graspers, tension is then applied to the graspers 26 to withdraw the pinches of acquired tissue through the window as described in prior embodiments. In this embodiment, the grasper shafts may extend only through the window, or the stapler head 12 might include a plate (similar to the pivot member 38) having guide holes for receiving the shafts of the graspers.

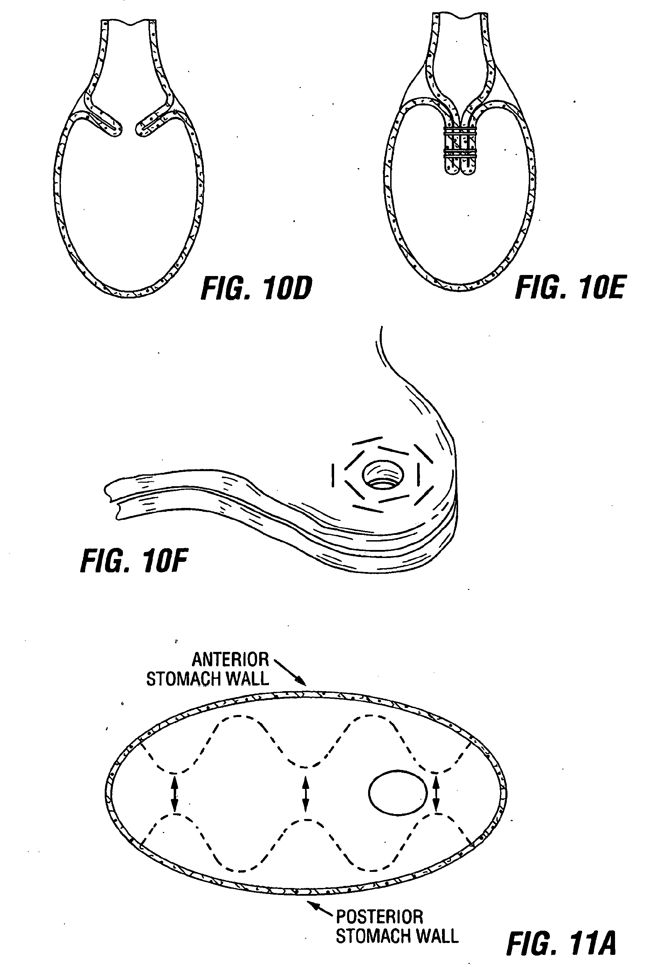

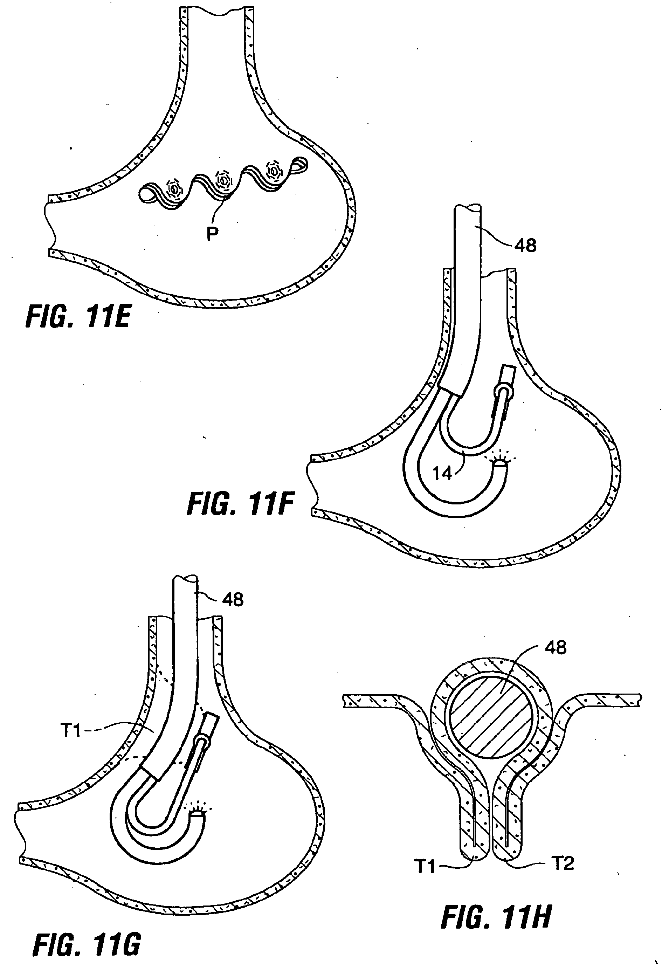

According to the disclosed embodiments, the tissue acquired for stapling can be tissue accessible by the graspers while the stapler head remains in a fixed position, or it can be from distinctly different areas of the organ. This latter technique may require acquiring tissue from one area of an organ or target tissue in one grasper, moving the stapler head to another target area and then acquiring second tissue for stapling from said target area. For example one area of tissue may be from the posterior side of the stomach and it may be anchored to tissue on the anterior side as illustrated in Fig. 11A. Figs. 10B - 10D illustrate that the pinches of tissue forming plications may have a number of different relative orientations. For example, Figs. 10B and 10C shows that the plications may be formed by attaching pinches P of tissue pulled towards one another such that the apexes of the folds formed by each pinch extend in opposite directions. Figs. 10C - 10E show that the pinches of tissue may instead be pulled in the same direction such that the apexes of each tissue pinch are more or less aligned with one another.

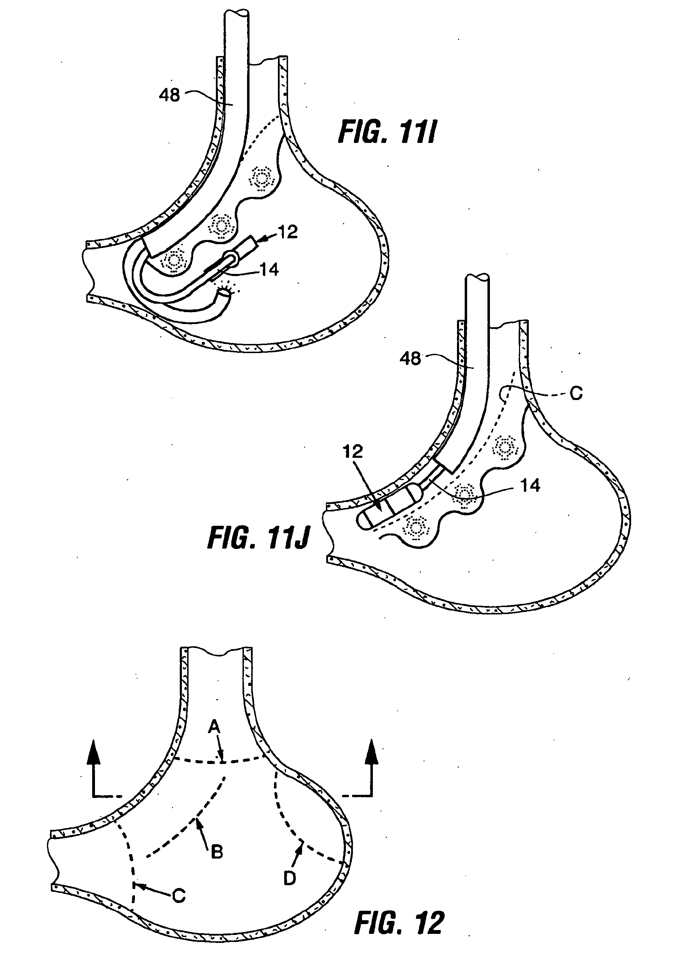

Devices using the disclosed principles may be used to form a single plication (Fig. 10A) within the stomach, or the device might be fired multiple times to create a line of plications partitioning the stomach in the desired configuration and location. Some of these locations, which are identified by letters A - D in Fig. 12, include, but are not limited to horizontal at the GE junction (A) partially across the stomach, vertical along the lesser curvature (B), transverse across the antrum (C) and transverse across the fundus (D).

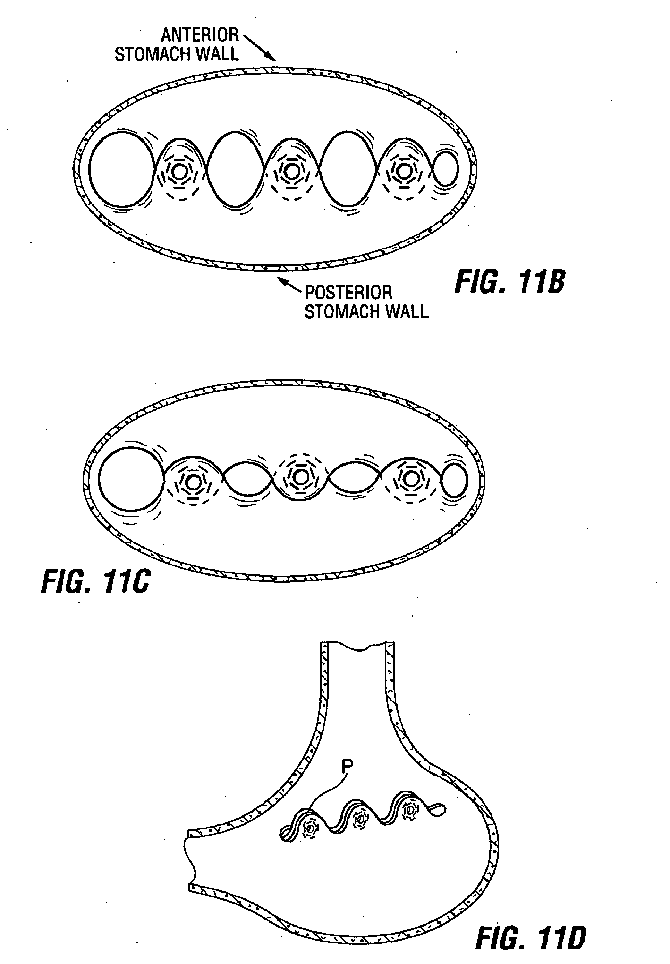

The distance between adjacent plications in a partition can be selected to allow gaps between each plication or to tightly space the placations to eliminate gaps virtually all together. The arrangement of the tissue pinches in each plication can be selected to give desired properties to the plication. For example, Fig. 11B shows a partition formed of three plications, where each plication is formed such that the tissue pinch from the anterior wall is positioned under the pinch from the posterior wall, whereas in Fig. 11C the plications are alternated such that in the center plication the pinch from the posterior wall is on top of the pinch from the anterior wall. Also, as shown in Figs. 11D and 11E, partitions may be formed using plications of the type disclosed above in connection with Fig. 10C, in which the pinches of tissue forming each plication have a common orientation.

Figs. 11F - 11J show an alternative exemplary partitioning method in which the guide tube 48 that receives the partitioning tool 10 (or, alternatively, the shaft 14 itself) is used as a guide for formation of the plications. Once the stapler head is in position within the stomach, the graspers (not shown) are used to acquire areas of tissue on opposite sides of the guide tube 48, such that when the acquired tissue is drawn through the window, it wraps partially around the guide tube as shown in Figs.11G - 11H. Compression and stapling are performed as described above to secure the pinches T1, T2 to one another. The stapler head may be repositioned multiple times to form several such plications as shown in Fig. 11I. Afterwards, the partitioning tool 10 is straightened and withdrawn, leaving the stomach partitioned to form a chute C (Fig. 11J) within the stomach.

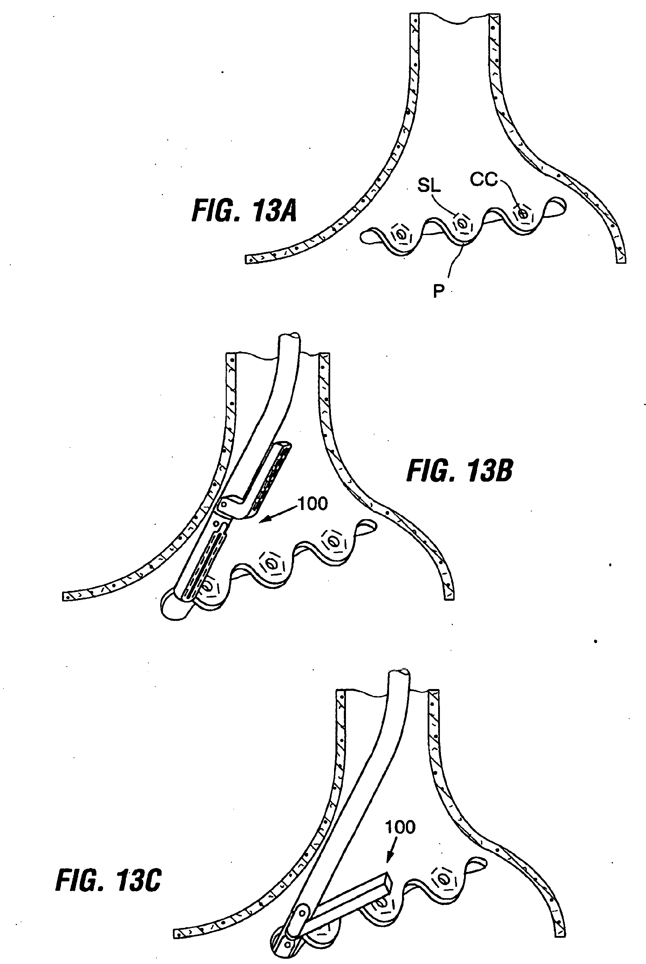

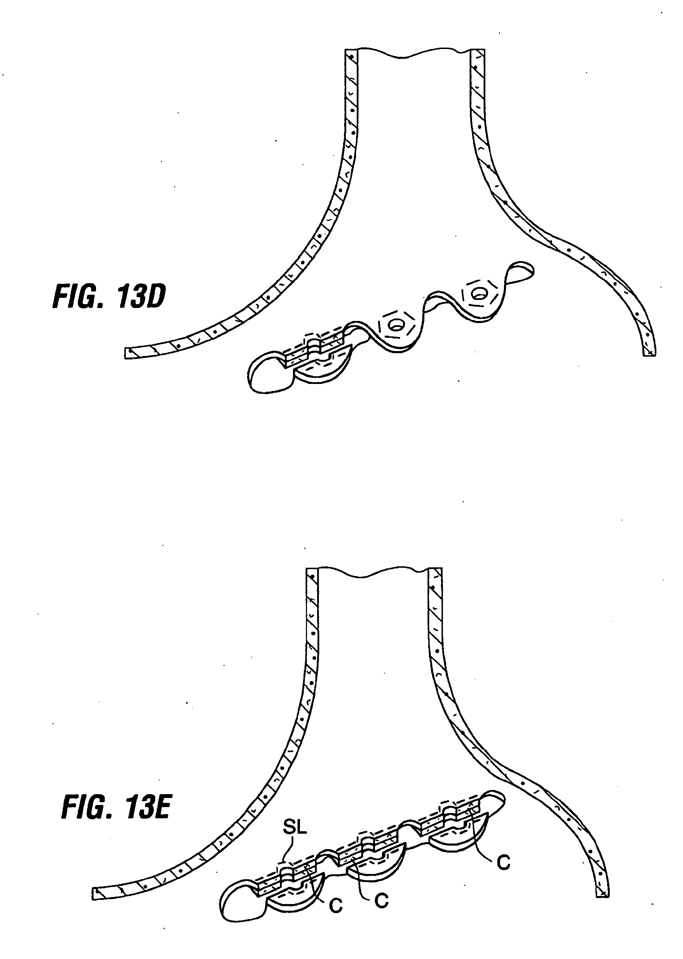

The partitions formed as described above may be reversed if at some point it is determined that it would be beneficial to do so. Referring to Figs. 13A through 13E, a partition can be reversed utilizing a conventional linear stapler 100 of a type that applies parallel rows of staples while forming a central cut between the staple rows. Referring to Fig. 13B, the stapler 100 is inserted through gap in or adjacent to the partition formed by plications P. For example, if the partition serves to define a narrow exit orifice for flow of food from the proximal stomach to the distal stomach, the stapler is inserted into the exit orifice and clamped across one or more of the plications (Fig. 13C). The stapler is activated and plications are separated by forming cuts C and forming staple lines SL. The process is repeated until the entire "partition" or collection of partitions are separated (Fig. 13D), restoring the natural geometry of the stomach (Fig. 13E).

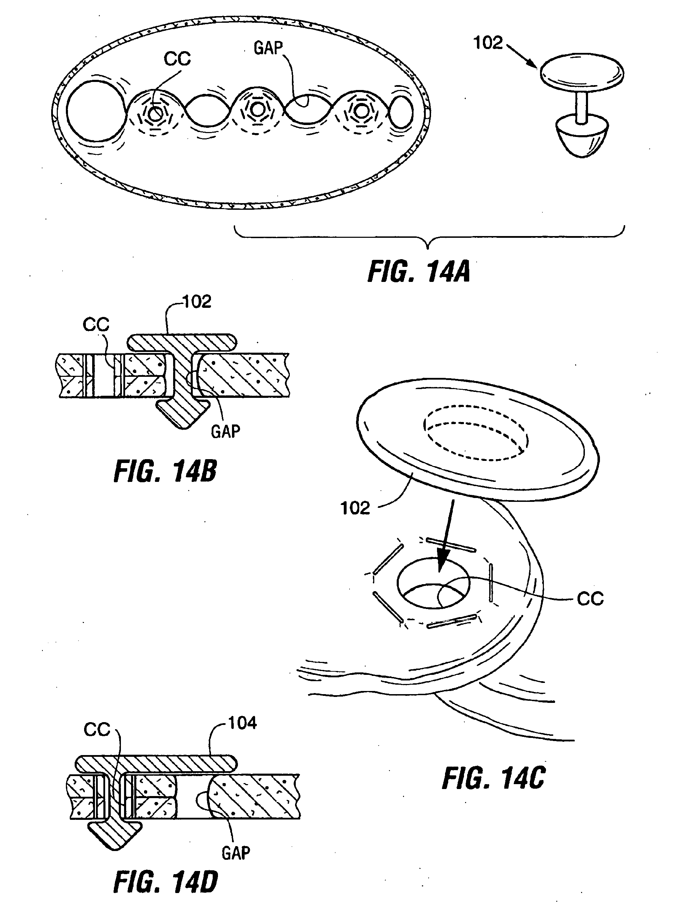

Partitions formed using the disclosed exemplary methods may be enhanced using plugs or pledgets. Plugs or pledgets 102 (Fig. 14A) can be inserted into the gaps between plications to increase the amount of restriction to flow of ingested food provided by the partition. Additionally, each plication can have a pledget/plug inserted into the hole cut into the plication (Fig. 14C) to ensure robust edge to edge healing of tissue; the pledget can be permanent or transitory in nature (e.g. biodegradable/bioerodible). The pledget can have a configuration on the top of the pledget which helps separate the newly created portions of the stomach as well, and can be modified to allow more or less food passage through gaps between plications. (Figure 14B). In one embodiment, a plug placed in the hole cut into the plication has an overlapping flange 104 that will extend to cover adjacent gaps between the plications.

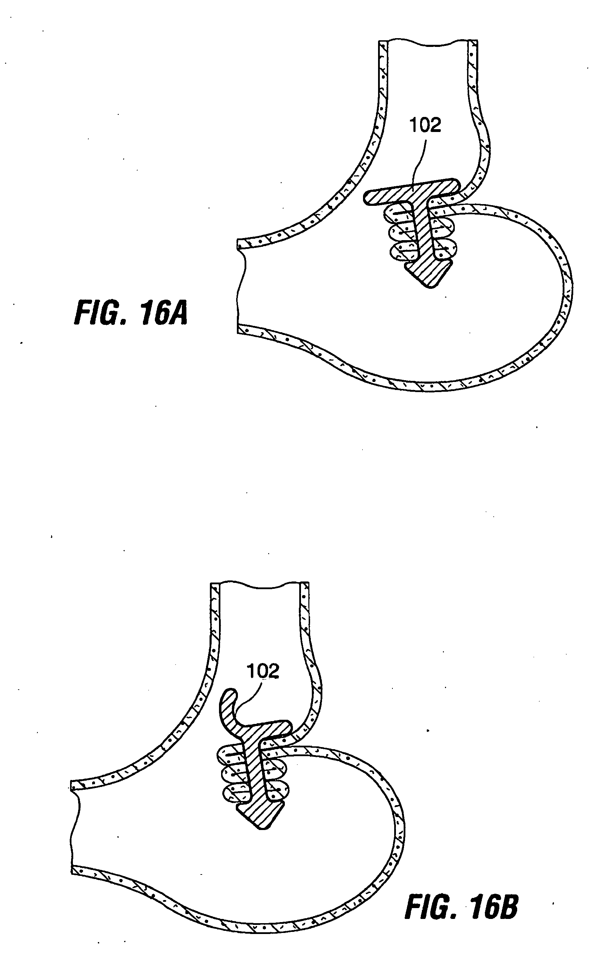

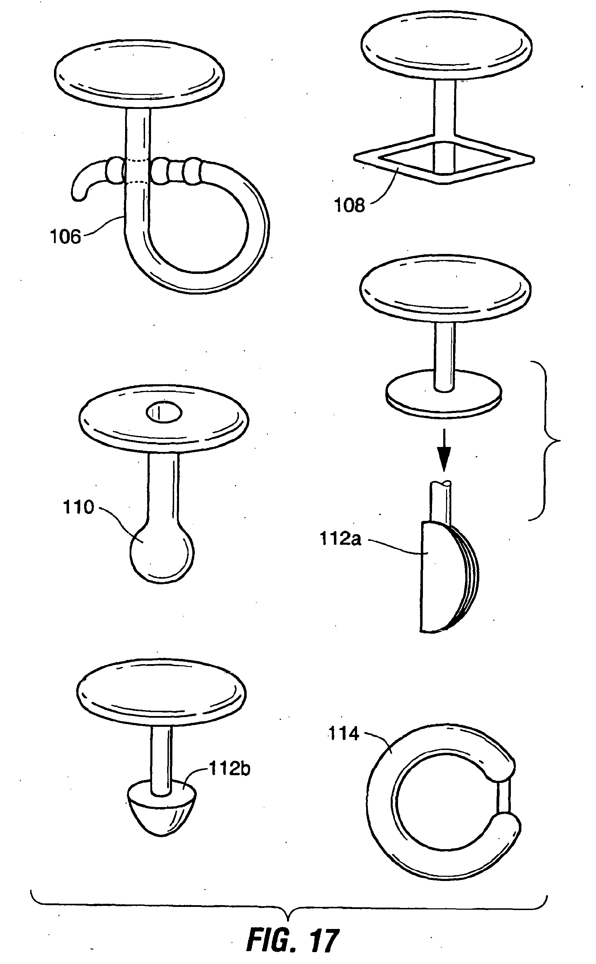

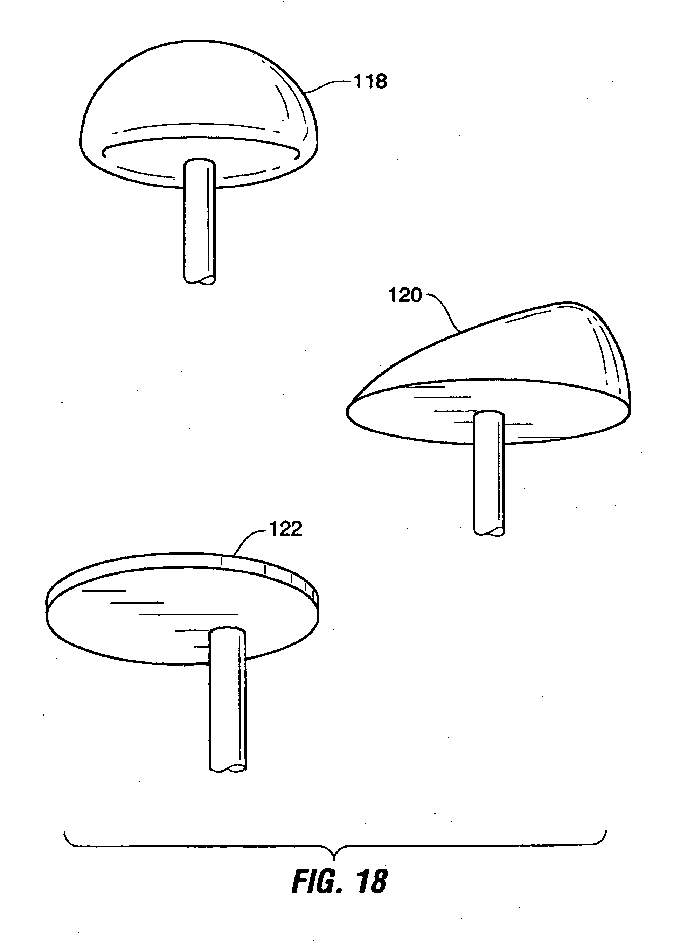

Figs. 15A through 15D illustrate that plugs/pledgets within the cut holes may be used to hold two or more two-layer plications together. For example, rather than joining two pinches of tissue as disclosed above to form a four-layer plication, the stapler may be used to separately staple and cut each pinch, forming a plurality of two-layer plications as shown in Fig. 15A. Afterwards, pairs (or larger groups) of the two-layer plications may be joined together to position the cut holes into alignment, and the plugs/pledgets may be inserted through the aligned holes to retain the plications as shown in Fig. 15B. Figs. 16A and 16B illustrate that plugs/pledgets 102 passed through the hole in one or more two- or four-layer plication can function as restrictive devices themselves, and be used to restrict flow of food towards the distal stomach. Various types of plugs/anchors are shown in Fig. 17 and Fig. 18.

Referring to Fig. 17, plugs/anchors may have fasteners 106 similar to zip ties, or moly-bolt type anchors 108. Other plugs/anchors may have inflatable portions 110 to anchor them in place (inflatable using air, liquid, or solids such as granular elements or miniature bearings. Still other plugs may have expandable anchors 112a, 112b that are insertable through the cut hole in the plication in a folded or collapsed shape, and that expand when released. In other embodiments, a locking ring 114 having a catch 116 may be opened into a "c", looped through the cut hole in the plication and then engaged at the catch to form a ring.

For plugs/anchors that have "lids" to prevent flow of material through a nearby hole cut into the plications, various lid designs may be used as shown in Fig. 18. These include the bulbous lid 118 which might also serve as a gastric space occupier, a tapered lid 120 that facilitates shedding of fluid and food material, an off-set lid 122 for restricting a tissue food orifice. Large lids can be used to facilitate sealing of a sectioned stomach. Ideally, the plug is manufactured out of a very compliant material (e.g. silicone, sartoprene, urethane, etc) which maintains alignment of holds but allows for movement of the stomach wall plications relative to one another.

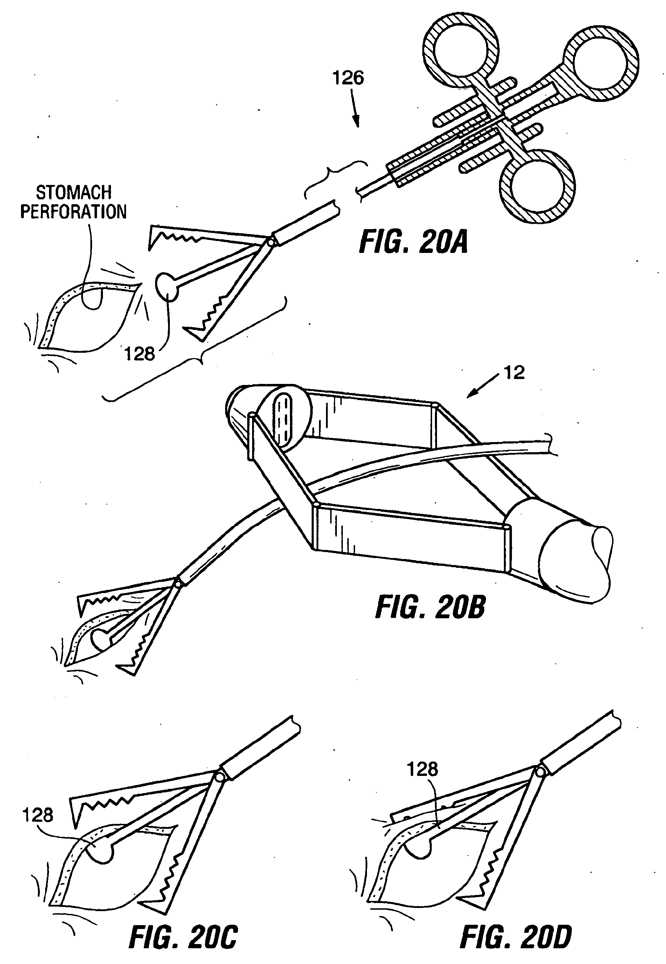

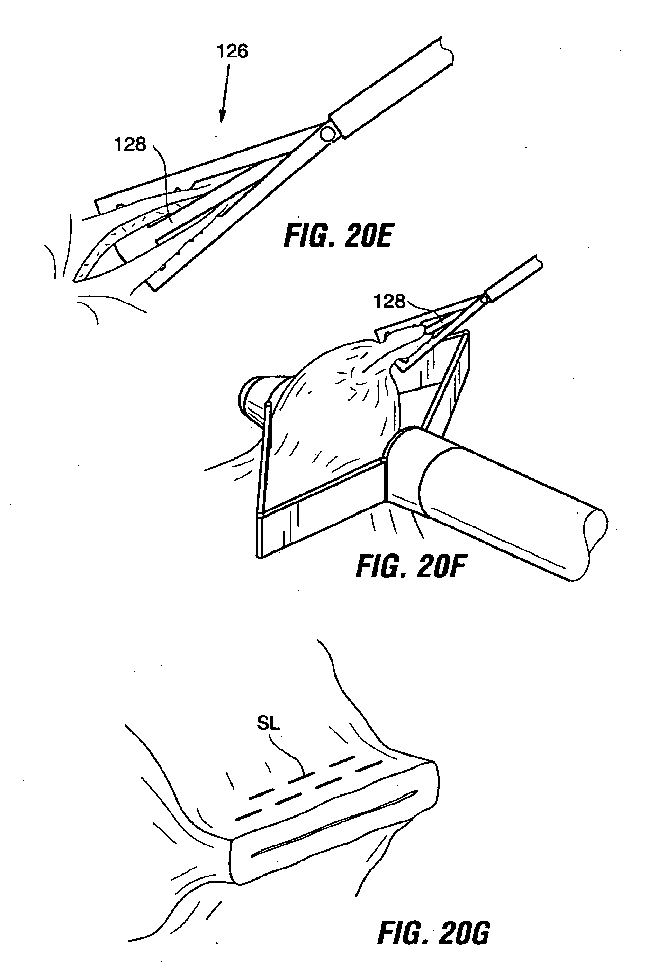

As discussed above, the tissue graspers can utilize any of a number of means for acquiring tissue including but not limited to vacuum, hooks, cork screws, or combinations of the above. In an alternate embodiment, the graspers 126 may have a dual action which is helpful in closure of a perforation of a tissue wall, such as the stomach or other organ. As shown in Fig. 10A, the graspers have a central "tongue" with graspers on each side. This embodiment allows each side of a perforation to be grasped independently and pulled between the stapler cartridge and anvil to ensure that the perforation is adequately closed. More specifically, the grasper is extended through the window in the stapler head as shown in Fig. 20B, and positioned with the tongue of the grasper within the perforation. The tongue is pivoted towards one jaw of the grasper as shown in Fig. 20D, to pinch one edge of the perforation between the tongue and that jaw. Next, as shown in 20E, the second jaw is moved towards the tongue to pinch the other edge of the perforation between the tongue and second jaw. The grasper is withdrawn, pulling the engaged tissue through the stapler window. The stapler is compressed to form a two-layer plication in the tissue with a row of staples SL extending through the plication. See Figs. 20F and 20G.

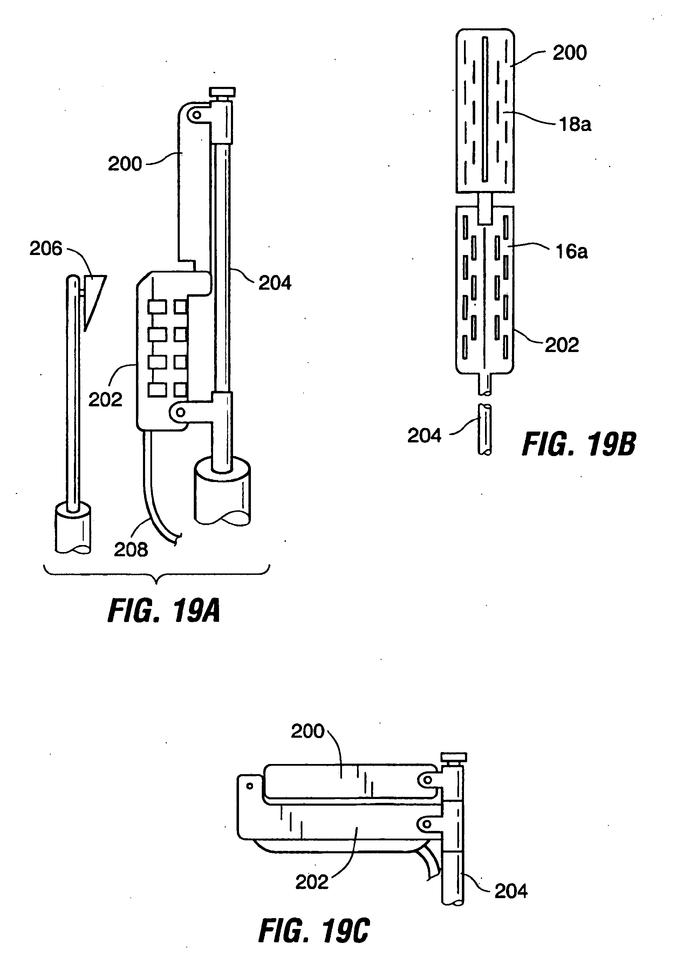

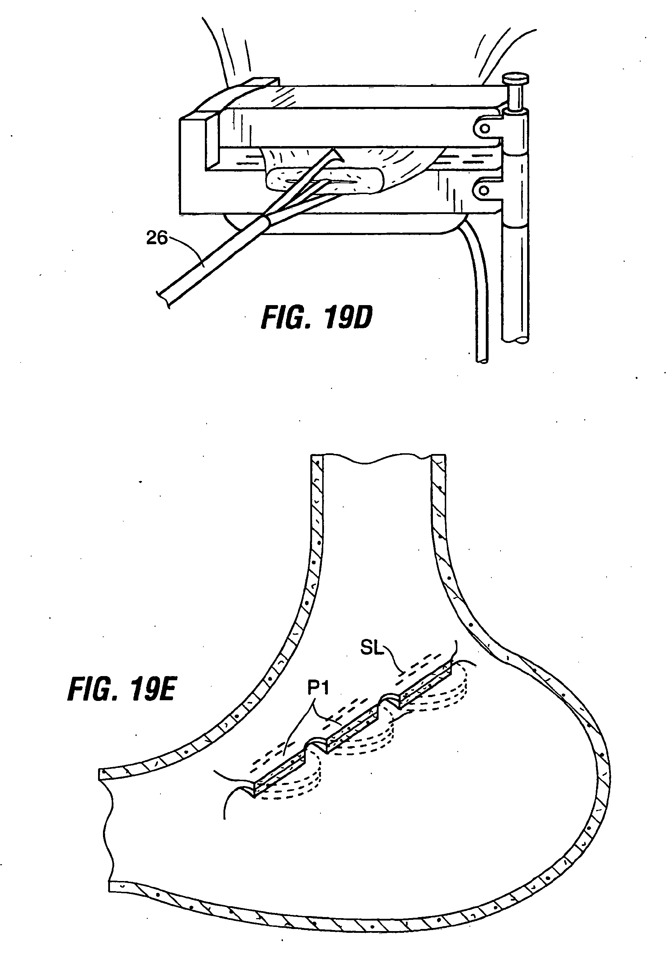

In an alternative stapler design shown in Figs. 19A - 19C, the cartridge 18a and anvil 16a as positioned on jaw members 200, 202 slidably positioned on a rod 204. A grasper 26 is used to draw tissue between the cartridge and anvil, and the cartridge and anvil are closed by advancing the jaw carrying the cartridge along the rod, thereby moving the jaws into the closed position (Fig. 19C) and compressing the tissue (Fig. 19D.. The tissue is stapled to produce a linear staple line. The tissue may additionally be cut by a blade 206 that is driven through the staple head. Hydraulic fluid driven through cable 208 is employed to drive the staples and may also be used to advance blade 206. With the variety of mechanisms and combinations possible, the device would be capable of excising intussusceptions, removing polyps, close perforations (holes) of the stomach or other body tissue, resolution of internal or external hemorrhoids, ulcers, perform tubal ligations, remove cervical lesions, produce pyloric tightening, and perform the removal of organs or tissue outside the GI tract. Referring to Fig. 19E, this stapler design may be used to form a plurality of plications P1 which have had the "lips" or apexes of the plications cut off by the stapling element. Staple line SL maintains apposition of the plicated tissue.

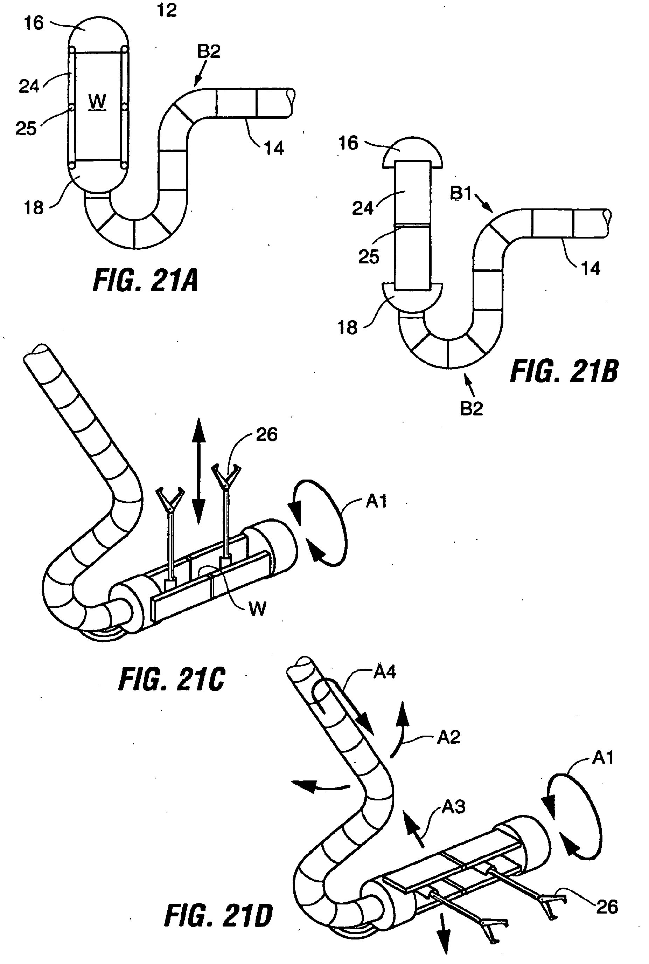

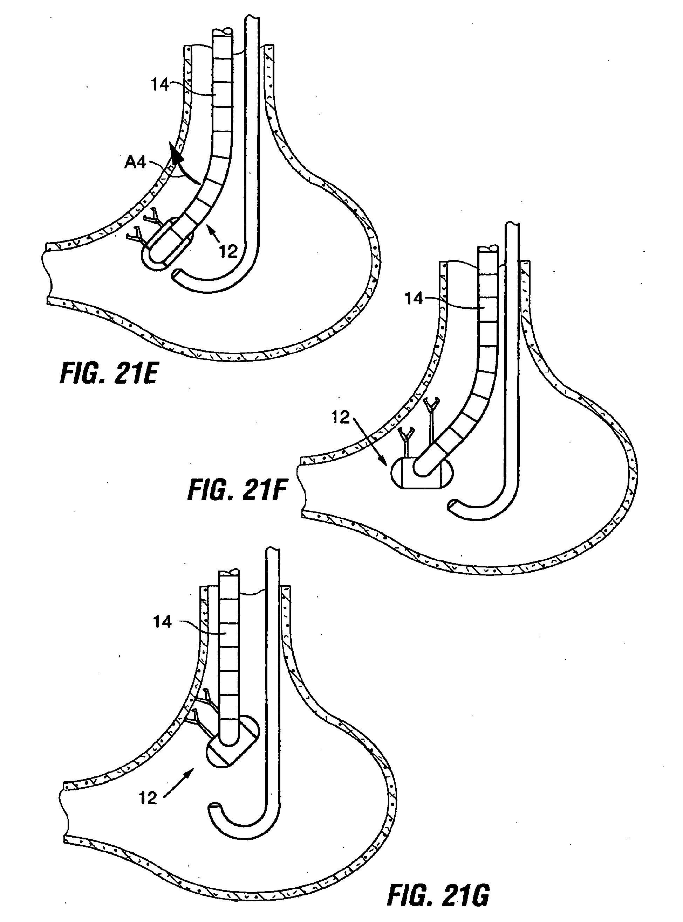

The partitioning tools described herein 10 may include a number of features that allow the stapler head 12 to be oriented as needed to ensure that the tissue pinches drawn into the window are properly aligned with one another and with the staple housing and anvil for optimal compression and stapling of the targeted tissue. As discussed, the head 12 may be articulatable in one or more directions using pull cables or other appropriate methods. Referring to Fig. 21a, shaft 14 may formable using into a predetermined shape using locking spine technology, to give the shaft an operative end having one or multiple bends, such as bend B1 and bend B1. Bends B1 and B2 may be within a single plane, or bend B2 may be within the plane of the straight section of the shaft while bend B1 extends out of the plane shared by B2 and the shaft. Additionally, the head 12 may be rotatable relative to its longitudinal axis by a wrist-type joint coupling the head 12 to the shaft 14. For example, see Fig. 21A in which the opening to window faces perpendicular to the shaft 14, whereas in Fig. 21B the window faces the shaft 14. Arrow A1 in Figs. 21C and 21C represents rotation of the head 12 relative to the shaft 14. Further, the shaft may be articulatable at the bends or other locations to allow adjustment of the head orientation, as indicated by arrows A2 (lateral articulation relative to the longitudinal section of shaft 14), arrows A3 (articulation towards/away from the longitudinal section of the shaft 14), and arrows A4 (articulation into/out of the page in a plane shared by the longitudinal section of the shaft, as also shown in Fig. 21E. Fig. 21F shows this same articulation but with the stapler head rotated to a different orientation to give access to a different area of the stomach wall. In the illustrated embodiment, during articulation in directions A2 and A3 the bends of the shaft remain within a single plane, which is the plane occupied by the longitudinal section of the shaft. Fig. 21G illustrates the shaft articulated in directions A2 and A3, but not in direction A4, such that the entire shaft is disposed within the plane of its longitudinal section.

In these drawings, only the ends of the graspers are shown for purposes of clarity.

In another embodiment, the staple head may be both articulatable and moveable into a laterally-offset position relative to its shaft.

It should be recognized that a number of variations of the above-identified embodiments will be obvious to one of ordinary skill in the art in view of the foregoing description. Moreover, features of the disclosed embodiments may be combined with one another and with other features (including those taught in the prior applications referenced herein) in varying ways to produce additional embodiments. Accordingly, the invention is not to be limited by those specific embodiments of the present invention as claimed, shown and described herein. The applications and exemplary methods listed are not limited to the treatment of diseases or procedures listed. Modifications of the above described tools and variations of this invention that are obvious to those of skill in the art are intended to be within the scope of this disclosure.

In one example, a method of forming a tissue structure in body tissue is described. The method comprises the steps of introducing a head having a first member and a second member into a body cavity; using a first tissue engager to engage a first pinch of body tissue within the body cavity; using a second tissue engager to engage a second pinch of body tissue within the body cavity; withdrawing the first tissue engager and the second tissue engager to withdraw the first and second pinches between the first and second members; compressing at least a portion of the first pinch and at least a portion of the second pinch between the first and second members; and advancing a fastener from the first member through the first and second pinches to form a tissue structure. In an example, advancing a fastener includes advancing a staple from the first member through the first and second pinches. The method may further include the step of forming a cut through the first and second pinches. Passing the first tissue engager may include advancing the first tissue engager from the head. The method may also include passing the first tissue engager between the first and second members prior to using the first engager to engage the first pinch, and passing the second tissue engager between the first and second members prior to using second tissue engager to engage the second pinch. The first and second tissue engagers may include shafts having proximal ends. In this example, the method includes: passing the head over the proximal ends of the first and second tissue engagers; and advancing the head over the shafts into the body cavity. The method further includes, after engaging the first and second pinches, advancing the head over the shafts into proximity with the first and second pinches, and with the head in proximity with the first and second pinches, the first tissue engager and the second tissue engager are withdrawn to draw the first and second pinches between the first and second members.

In another example, the method further includes the step of positioning a partition between the first and second pinches, wherein compressing at least a portion of the first pinch and at least a portion of the second pinch between the first and second members is performed with the partition between the first and second pinches. The method may include using at least two first tissue engagers to engage the first pinch of body tissue, and withdrawing the at least two first tissue engagers to withdraw the first pinch between the first and second members.

In another example, a method of closing a hole in body tissue, comprising the steps of: providing a grasper including a first jaw member, a second jaw member, and a third member between the first and second jaw members; advancing the grasper to a hole in body tissue; engaging a first edge of the hole between the first jaw member and the third member; engaging a second edge of the hole between the second jaw member and the third member; with the first and second edges of the hole engaged by the grasper, manipulating the grasper to draw the first and second edges and an adjacent pinch of tissue between first and second elements of a fastening tool; compressing the pinch of tissue between the first and second elements of the fastening tool; and advancing a fastener from the first element through the pinch of tissue is contemplated.

## Claims

1. A system for forming a tissue structure in body tissue, the system comprising:
an elongate shaft having a distal end;
a head (12) positioned on the distal end of the elongate shaft, the head having a first member (18) and a second member (16);
an arm assembly (24) having a first section pivotally coupled to the first member and a second section pivotally coupled to the second member such that movement of the arm assembly to an expanded position moves the first and second members relatively towards one another and increases the lateral dimension of an expandable window bounded by the arm assembly and the first and second members;
a first tissue engager (26) extendable between the first and second members to engage a first pinch (T1) of body tissue within the body cavity and retractable to withdraw the first pinch between the first and second members through the expandable window;
a second tissue engager (26) extendable between the first and second members to engage a second pinch (T2) of body tissue within the body cavity and retractable to withdraw the second pinch between the first and second members adjacent to the first pinch of body tissue through the expandable window, wherein the first and second members are moveable relatively towards one another into a tissue compression position in which the first and second members are positioned to compress at least a portion of the first pinch and at least a portion of the second pinch between the first and second members; and
at least one fastener (20) advanceable from the first member through the first and second pinches.

2. The system according to claim 1, wherein the first engager includes a forceps (26).

3. The system according to claim 1, wherein the first engager includes a vacuum device (28).

4. The system according to claim 1 wherein the first engager includes a helical piercing element (30a, 30b).

5. The system according to claim 1 wherein the first engager includes a hook (32).

6. The system according to claim 1, further include a guide tube (34) coupled to the head, wherein the first engager is slidable within the guide tube.

7. The system according to claim 1, wherein the first engager includes a pair of engagers.

8. The system according to claim 1 wherein the first engager includes an Alice-type grasper (33).

9. The system according to claim 1 wherein each of the first and second engagers is advanceable from a first position on a first side of the head, between the first and second members, to a second position on a second side of the head, and retractable from the second position to the first position to withdraw tissue between the first and second members.

10. The system according to claim 9, wherein the first and second engagers are advanceable between the first and second positions along non-parallel paths.

11. The system according to claim 9, wherein the first and second engagers are advanceable between the first and second positions along parallel paths.

12. The system according to claim 1, wherein the at least one fastener is a circular array of fasteners.

13. The system according to claim 12, wherein the at least one fastener is a circular array of stapled plications, the system further comprising:
a cutter (22) for cutting a hole through the stapled plications within the circular array of staples.

14. The system according to claim 13, further comprising:
a plug dimensioned to be inserted into the hole in the stapled plication, the plug having an anchor to retain the plug through the plication.

## Patentansprüche

1. System zum Formen einer Gewebestruktur in Körpergewebe, wobei das System aufweist:
einen länglichen Schaft mit einem distalen Ende;
einem Kopf (12), der am distalen Ende des länglichen Schafts angeordnet ist, wobei der Kopf ein erstes Element (18) und ein zweites Element (16) aufweist;
eine Armanordnung (24) mit einem ersten Abschnitt, der schwenkbar mit dem ersten Element gekoppelt ist, und einem zweiten Abschnitt, der schwenkbar mit dem zweiten Element gekoppelt ist, so dass eine Bewegung der Armanordnung zu einer ausgedehnten Position das erste und zweite Element relativ zueinander hin bewegt und die laterale Abmessung eines ausdehnbaren Fensters vergrößert, das durch die Armanordnung und das erste und zweite Element begrenzt ist;
eine erste Gewebeeingriffsvorrichtung (26), die zwischen dem ersten und zweiten Element ausdehnbar ist, um eine erste Einschnürung (T1) des Körpergewebes innerhalb des Körperhohlraums in Eingriff zu nehmen, und einziehbar ist, um die erste Einschnürung zwischen dem ersten und zweiten Element durch das ausdehnbare Fenster zurückzuziehen;
eine zweite Gewebeeingriffsvorrichtung (26), die zwischen dem ersten und zweiten Element ausdehnbar ist, um eine zweite Einschnürung (T2) des Körpergewebes innerhalb des Körperhohlraums in Eingriff zu nehmen, und einziehbar ist, um die zweite Einschnürung zwischen dem ersten und zweiten Element benachbart zur ersten Einschnürung des Körpergewebes durch das ausdehnbare Fenster zurückzuziehen, wobei das erste und zweite Element relativ zueinander in eine Gewebekompressionsposition beweglich sind, in der das erste und zweite Element so angeordnet sind, dass sie mindestens einen Abschnitt der ersten Einschnürung und mindestens einen Abschnitt der zweiten Einschnürung zwischen dem ersten und zweiten Element komprimieren; und
mindestens ein Befestigungselement (20), das vom ersten Element durch die erste und zweite Einschnürung vorschiebbar ist.

2. System nach Anspruch 1, wobei die erste Eingriffsvorrichtung eine Zange (26) aufweist.

3. System nach Anspruch 1, wobei die erste Eingriffsvorrichtung eine Vakuumvorrichtung (28) aufweist.

4. System nach Anspruch 1 wobei die erste Eingriffsvorrichtung ein spiralförmiges Stechelement (30a, 30b) aufweist.

5. System nach Anspruch 1 wobei die erste Eingriffsvorrichtung einen Haken (32) aufweist.

6. System nach Anspruch 1, das ferner eine Führungsröhre (34) aufweist, die mit dem Kopf gekoppelt ist, wobei die erste Eingriffsvorrichtung innerhalb der Führungsröhre verschiebbar ist.

7. System nach Anspruch 1, wobei die erste Eingriffsvorrichtung ein Paar Eingriffsvorrichtungen aufweist.

8. System nach Anspruch 1 wobei die erste Eingriffsvorrichtung einen Alice-Greifer (33) aufweist.

9. System nach Anspruch 1 wobei jede der ersten und zweiten Eingriffsvorrichtung von einer ersten Position auf einer ersten Seite des Kopfes zwischen dem ersten und zweiten Element zu einer zweiten Position auf einer zweiten Seite des Kopfes vorschiebbar ist, und von der zweiten Position zur ersten Position einziehbar ist, um Gewebe zwischen dem ersten und zweiten Element zurückzuziehen.

10. System nach Anspruch 9, wobei die erste und zweite Eingriffsvorrichtung zwischen der ersten und zweiten Position längs nichtparalleler Wege vorschiebbar sind.

11. System nach Anspruch 9, wobei die erste und zweite Eingriffsvorrichtung zwischen der ersten und zweiten Position längs paralleler Wege vorschiebbar sind.

12. System nach Anspruch 1, wobei das mindestens eine Befestigungselement eine kreisförmige Anordnung von Befestigungselementen ist.

13. System nach Anspruch 12, wobei das mindestens eine Befestigungselement eine kreisförmige Anordnung von geklammerten Plikaturen ist, wobei das System ferner aufweist:
eine Schneidvorrichtung (22) zum Schneiden eines Lochs durch die geklammerten Plikaturen innerhalb der kreisförmigen Anordnung von Plikaturen.

14. System nach Anspruch 13, das ferner aufweist:
einen Stopfen, der bemessen ist, in das Loch in der geklammerten Plikatur eingesetzt zu werden, wobei der Stopfen eine Verankerung aufweist, um den Stopfen durch die Plikatur zu halten.

## Revendications

1. Système permettant de former une structure tissulaire dans un tissu corporel, ledit système comprenant :
une tige oblongue ayant une extrémité distale ;
une tête (12) positionnée à l'extrémité distale de la tige oblongue, ladite tête comportant un premier élément (18) et un deuxième élément (16) ;
un ensemble de bras (24) comportant un premier segment accouplé de manière pivotante au premier élément et un deuxième segment accouplé de manière pivotante au deuxième élément, de telle manière qu'un actionnement de l'ensemble de bras vers une position d'expansion entraîne un déplacement relatif du premier et du deuxième éléments l'un vers l'autre et augmente la dimension latérale d'une fenêtre expansible délimitée par l'ensemble de bras et par le premier et le deuxième éléments ;
un premier inserteur de tissu (26) extensible entre le premier et le deuxième éléments pour insérer une première pincée (T1) de tissu corporel à l'intérieur de la cavité corporelle, et rétractable pour retirer la première pincée entre le premier et le deuxième éléments au travers de la fenêtre expansible ;
un deuxième inserteur de tissu (26) extensible entre le premier et le deuxième éléments pour insérer une deuxième pincée (T2) de tissu corporel à l'intérieur de la cavité corporelle, et rétractable pour retirer la deuxième pincée de manière adjacente à la première pincée de tissu corporel entre le premier et le deuxième éléments au travers de la fenêtre expansible, le premier et le deuxième éléments étant mobiles relativement l'un vers l'autre dans une position de compression de tissu où le premier et le deuxième éléments sont positionnés pour comprimer au moins une partie de la première pincée et au moins une partie de la deuxième pincée entre le premier et le deuxième éléments ; et
au moins une attache (20) pouvant être avancée par le premier élément au travers de la première et de la deuxième pincées.

2. Système selon la revendication 1, où le premier inserteur est pourvu d'un forceps (26).

3. Système selon la revendication 1, où le premier inserteur est pourvu d'un dispositif d'aspiration (28).

4. Système selon la revendication 1, où le premier inserteur est pourvu d'un élément de perçage hélicoïdal (30a, 30b).

5. Système selon la revendication 1, où le premier inserteur est pourvu d'un crochet (32).

6. Système selon la revendication 1, comprenant en outre un tube de guidage (34) accouplé à la tête, ledit premier inserteur étant coulissant à l'intérieur du tube de guidage.

7. Système selon la revendication 1, où le premier inserteur est pourvu d'une paire d'inserteurs.

8. Système selon la revendication 1, où le premier inserteur est pourvu d'une pince Alice (33).

9. Système selon la revendication 1, où le premier ainsi que le deuxième inserteurs peuvent être avancés d'une première position sur un premier côté de la tête, entre le premier et le deuxième éléments, vers une deuxième position sur un deuxième côté de la tête, et sont rétractable de la deuxième position vers la première position pour retirer le tissu entre le premier et le deuxième éléments.

10. Système selon la revendication 9, où le premier et le deuxième inserteurs peuvent être avancés entre la première et la deuxième positions sur des courses non parallèles.

11. Système selon la revendication 9, où le premier et le deuxième inserteurs peuvent être avancés entre la première et la deuxième positions sur des courses parallèles.

12. Système selon la revendication 1, où le ou les attaches sont un alignement circulaire d'attaches.

13. Système selon la revendication 12, où le ou les attaches sont un alignement circulaire de plis agrafés, le système comprenant en outre :
une lame (22) permettant de découper un trou dans les plis agrafés à l'intérieur de l'alignement circulaire d'agrafes.

14. Système selon la revendication 13, comprenant en outre :
une fiche de dimensions prévues pour une insertion dans le trou du pli agrafé, ladite fiche étant pourvue d'un ancrage permettant son maintien dans le pli.
